# EUROPEAN PATENT SPECIFICATION

(11) **EP 0 876 402 B1**
(45) Date of publication and mention of the grant of the patent: **13.10.2004**
(21) Application number: 96945183.0
(22) Date of filing: 10.12.1996
(51) Int. Cl.: C07K 14/705, C07K 14/72, C07K 14/435, C07K 14/60, C07K 14/61, C07K 14/47, C12N 15/12, C12N 15/09, C12N 15/10, C12N 15/00, C12N 5/10

(54) **GROWTH HORMONE SECRETAGOGUE RECEPTOR FAMILY**
WACHSTUMSHORMONSEKRETIONSFÖRDERER REZEPTORFAMILIE
FAMILLE DE RECEPTEURS SECRETAGOGUES D'HORMONES DE CROISSANCE

(30) Priority: 13.12.1995 US 8582 P; 06.06.1996 US 18962 P
(43) Date of publication of application: 11.11.1998
(73) Proprietor: MERCK & CO., INC., Rahway, New Jersey 07065 (US)
(72) Inventor: ARENA, Joseph, P., Rahway, NJ 07065 (US); CULLY, Doris, F., Rahway, NJ 07065 (US); FEIGHNER, Scott, D., Rahway, NJ 07065 (US); HOWARD, Andrew, D., Rahway, NJ 07065 (US); LIBERATOR, Paul, A., Rahway, NJ 07065 (US); SCHAEFFER, James, M., Rahway, NJ 07065 (US); VAN DER PLOEG, Leonardus, Rahway, NJ 07065 (US)
(74) Representative: Horgan, James Michael Frederic
(86) International application number: PCT/US1996/019445
(87) International publication number: WO 1997/021730

(56) References cited:
- US-A- 5 057 417
- US-A- 5 583 010
- BLAKE A D ET AL: "DESENSITIZATION STUDIES USING PERIFUSED RAT PITUITARY CELLS SHOW THAT GROWTH HORMONE-RELEASING HORMONE AND HIS-D-TRP-ALA-TRP-D-PHE-LYS-NH2 STIMULATE GROWTH HORMONE RELEASE THROUGH DISTINCT RECEPTOR SITES" JOURNAL OF ENDOCRINOLOGY, BRISTOL, GB, vol. 129, no. 1, 1991, pages 11-19, XP001021833 ISSN: 0022-0795
- SETHUMADHAVAN K ET AL: "DEMONSTRATION AND CHARACTERIZATION OF THE SPECIFIC BINDING OF GROWTH HORMONE-RELEASING PEPTIDE TO RAT ANTERIOR PITUITARY AND HYPOTHALAMIC MEMBRANES" BIOCHEMICAL AND BIOPHYSICAL RESEARCH COMMUNICATIONS, ACADEMIC PRESS INC. ORLANDO, FL, US, vol. 178, no. 1, 15 July 1991 (1991-07-15), pages 31-37, XP001019190 ISSN: 0006-291X
- POMES A ET AL: "SOLUBILIZATION AND CHARACTERIZATION OF A GROWTH HORMONE SECRETAGOGUE RECEPTOR FROM PORCINE ANTERIOR PITUITARY MEMBRANES" BIOCHEMICAL AND BIOPHYSICAL RESEARCH COMMUNICATIONS, ACADEMIC PRESS INC. ORLANDO, FL, US, vol. 225, 1996, pages 939-945, XP001019261 ISSN: 0006-291X
- NARGUND RAVI P ET AL: "Novel probes derived from MK-0677 for characterization of the growth hormone secretagogue receptor." ABSTRACTS OF PAPERS AMERICAN CHEMICAL SOCIETY, vol. 212, no. 1-2, 1996, page MEDI 9 XP001018698 212th American Chemical Society National Meeting;Orlando, Florida, USA; August 25-29, 1996 ISSN: 0065-7727
- PONG S-S ET AL: "IDENTIFICATION OF A NEW G-PROTEIN-LINKED RECEPTOR FOR GROWTH HORMONE SECRETAGOGUES" MOLECULAR ENDOCRINOLOGY, BALTIMORE, MD, US, vol. 10, no. 1, 1996, pages 57-61, XP001018700 ISSN: 0888-8809
- HRENJUK D ET AL: "CYTOCHEMICAL IDENTIFICATION AND CLONING OF A G-PROTEIN COUPLED GROWTH HORMONE SECRETAGOGUE RECEPTOR" SOCIETY FOR NEUROSCIENCE ABSTRACTS, SOCIETY FOR NEUROSCIENCE, US, vol. 22, no. 1-3, 1996, page 1300 XP001018696 ISSN: 0190-5295
- SMITH R G ET AL: "MODULATION OF PULSATILE GH RELEASE THROUGH A NOVEL RECEPTOR IN HYPOTHALAMUS AND PITUITARY GLAND" RECENT PROGRESS IN HORMONE RESEARCH, ACADEMIC PRESS, NEW YORK, NY, US, vol. 51, 1996, pages 261-286, XP001018702 ISSN: 0079-9963
- DEAN D C ET AL: "DEVELOPMENT OF A HIGH SPECIFIC ACTIVITY SULFUR-35-LABELED SULFONAMIDE RADIOLIGAND THAT ALLOWED THE IDENTIFICATION OF A NEW GROWTH HORMONE SECRETAGOGUE RECEPTOR" JOURNAL OF MEDICINAL CHEMISTRY, AMERICAN CHEMICAL SOCIETY. WASHINGTON, US, vol. 39, no. 9, 1996, pages 1767-1770, XP001018701 ISSN: 0022-2623
- SCIENCE, 16 August 1996, Vol. 273, HOWARD et al., "A Receptor in Pituitary and Hypothalamus That Functions in Growth Hormone Release", pages 974-977.
- JOURNAL OF CLINICAL ENDOCRINOLOGY AND METABOLISM, October 1994, Vol. 79, No. 4, ALOI et al., "Neuroendocrine Responses to a Novel Growth Hormone Secretagogue, L-692,429, In Healthy Older Subjects", pages 943-949.
- JOURNAL OF CLINICAL ENDOCRINOLOGY AND METABOLISM, October 1994, Vol. 79, No. 4, BOWERS, C.Y., "Editorial: On a Peptidomimetic Growth Hormone-Releasing Peptide", pages 940-942.

## Description

### FIELD OF THE INVENTION

This invention relates to a new family of receptors, which includes the growth hormone secretagogue receptors (GHSRs) and growth hormone secretagogue-related receptors (GHSRRs), nucleic acids encoding these receptors; and to the use of a GHSR to identify growth hormone secretagogues and compounds that modulate GHSR function.

### BACKGROUND OF THE INVENTION

Growth hormone (GH) is an anabolic hormone capable of promoting linear growth, weight gain and whole body nitrogen retention. Classically, GH is thought to be released primarily from the somatotroph cells of the anterior pituitary under the coordinate regulation of two hypothalamic hormones, growth hormone releasing factor (GHRF or GRF) and somatostatin. Both GHRF stimulation and somatostatin inhibition of the release of GH occurs by the specific engagement of receptors on the cell membrane of the somatotroph.

Recent evidence has been mounting which suggests that GH release is also stimulated by a group of short peptides, the growth hormone releasing peptides (GHRP; GHRP-6, GHRP-2 [hexarelin]) which are described, for example, in U.S. Patent No. 4,411,890, PCT Patent Pub. No. WO 89/07110, PCT Patent Pub. No. WO 89/07111, PCT Patent Pub. No. WO 93/04081, and *J. Endocrinol Invest*., *15* (Suppl 4), 45 (1992). These peptides function by selectively binding to distinct somatotroph cell membrane receptor, the growth hormone secretagogue receptor(s) (GHSRs). A medicinal chemical approach has resulted in the design of several classes of orally-active, low molecular weight, non-peptidyl compounds which bind specifically to this receptor and result in the pulsatile release of GH. Such compounds possessing growth hormone secretagogue activity are disclosed, for example, in the following: U.S. Patent No. 3,239,345; U.S. Patent No. 4,036,979; U.S. Patent No. 4,411,890; U.S. Patent No. 5,206,235; U.S. Patent No. 5,283,241; U.S. Patent No. 5,284,841; U.S. Patent No. 5,310,737: U.S. Patent No. 5.317,017; U.S. Patent No. 5,374,721; U.S. Patent No. 5,430,144; U.S. Patent No. 5,434,261; U.S. Patent No. 5,438,136; U.S. Patent No. 5,494,919; U.S. Patent No. 5,494,920; U.S. Patent No. 5,492,916; EPO Patent Pub. No. 0,144,230; EPO Patent Pub. No. 0.513.974; PCT Patent Pub. No. WO 94/07486; PCT Patent Pub. No. WO 94/08583; PCT Patent Pub. No. WO 94/11012; PCT Patent Pub. No. WO 94/13696; PCT Patent Pub. No. WO 94/19367; PCT Patent Pub. No. WO 95/03289; PCT Patent Pub. No. WO 95/03290; PCT Patent Pub. No. WO 95/09633; PCT Patent Pub. No. WO 95/11029; PCT Patent Pub. No. WO 95/12598; PCT Patent Pub. No. WO 95/13069; PCT Patent Pub. No. WO 95/14666; PCT Patent Pub. No. WO 95/16675; PCT Patent Pub. No. WO 95/16692; PCT Patent Pub. No. WO 95/17422; PCT Patent Pub. No. WO 95/17423; PCT Patent Pub. No. WO 95/34311; PCT Patent Pub. No. WO 96/02530; *Science*, 260, 1640-1643 (June 11, 1993); *Ann. Rep. Med*. *Chem*., *28*, 177-186 (1993); *Bioorg*. *Med*. *Chem*. *Ltrs*., 4(22), 2709-2714 (1994); and *Proc. Natl*. *Acad*. *Sci*. *USA* 92, 7001-7005 (July 1995).

The use of orally-active agents which stimulate the pulsatile release of GH would be a significant advance in the treatment of growth hormone deficiency in children and adults as well as provide substantial benefit under circumstances where the anabolic effects of GH might be exploited clinically (e.g. post-hip fracture rehabilitation, the frail elderly and in post-operative recovery patients).

It would also be desirable to know the molecular structure of growth hormone secretagogue receptors in order to analyze this new receptor family and understand its normal physiological role in concert with the actions of GHRF and somatostatin. This could lead to a better understanding of the *in vivo* processes which occur upon ligand-receptor binding. Further, it would be desirable to use cloned-growth hormone secretagogue receptors as essential components of an assay system which can identify new growth hormone secretagogues.

### DETAILED DESCRIPTION OF THE INVENTION

This invention relates to a novel family of receptors which includes growth hormone secretagogue receptors (GHSRs) and growth hormone secretagogue-related receptors (GHSRRs).

A first aspect of this invention are the growth hormone secretagogue receptors, which are free from receptor associated proteins. GHSRs may be from any species, and in further embodiments may be isolated or purified. One embodiment of this invention is human growth hormone secretagogue receptor (hGHSR). free from receptor-associated proteins. A further aspect of this invention is hGHSR which is isolated or purified.

Another aspect of this invention is swine growth hormone secretagogue receptor (sGHSR), free from receptor-associated proteins. A further aspect of this invention is sGHSR which is isolated or purified.

Another aspect of this invention is rat growth hormone secretagogue receptor (rGHSR), free from receptor-associated proteins. A further aspect of this invention is rGHSR which is isolated or purified.

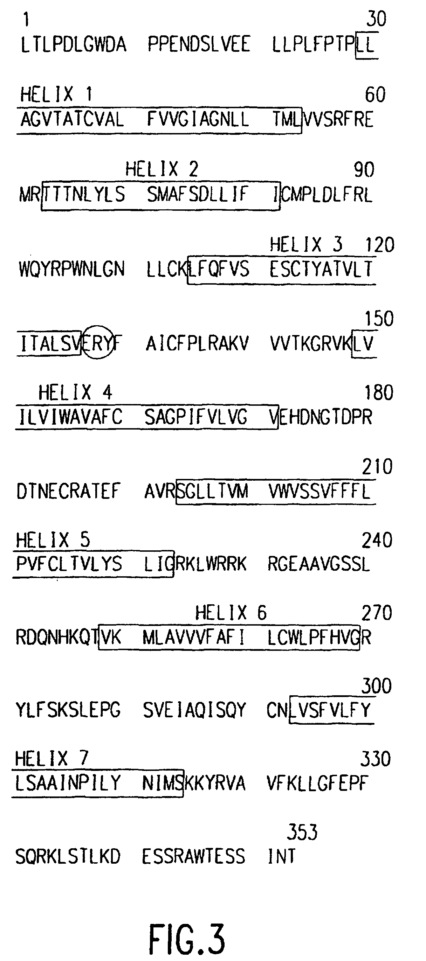

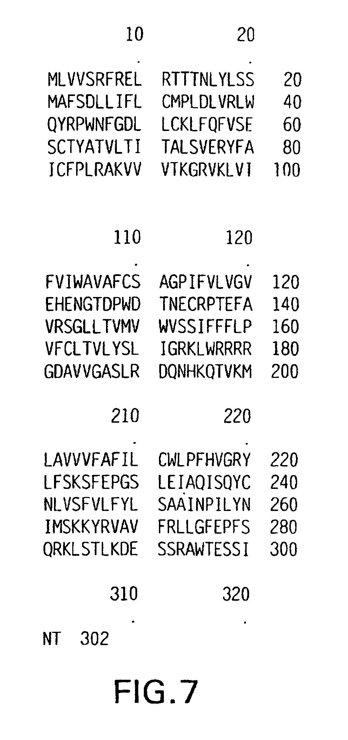

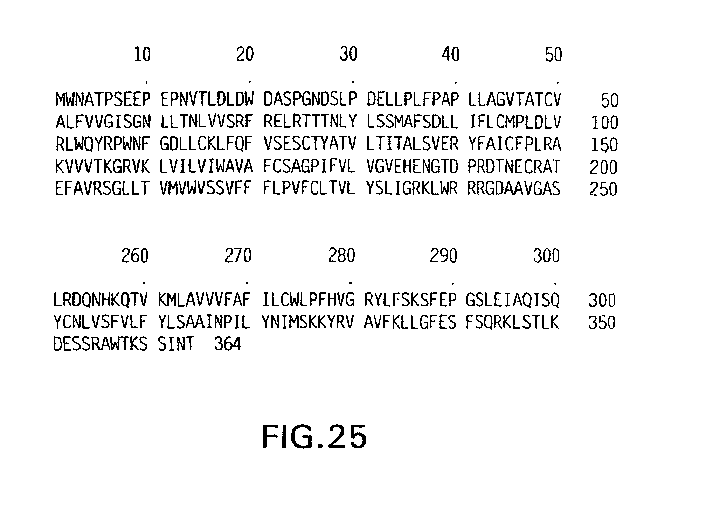

Thus the present invention provides an isolated growth hormone secretagogue receptor which comprises an amino acid sequence set forth in Figure 3, 7, 12, 22 or 25.

The present invention also provides a growth hormone secretagogue receptor, free from receptor-associated proteins, which comprises an amino acid sequence as set forth in Figure 3, 7, 12, 22 or 25.

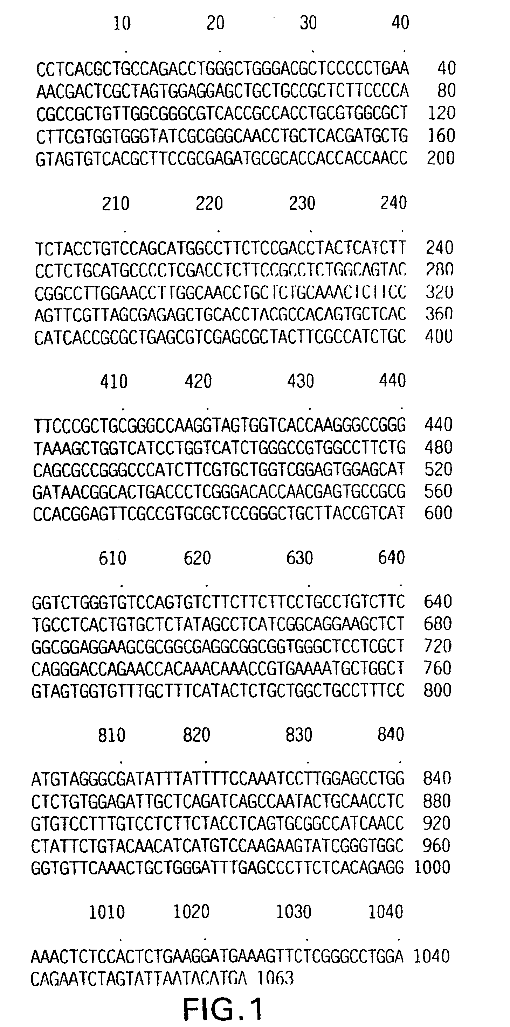

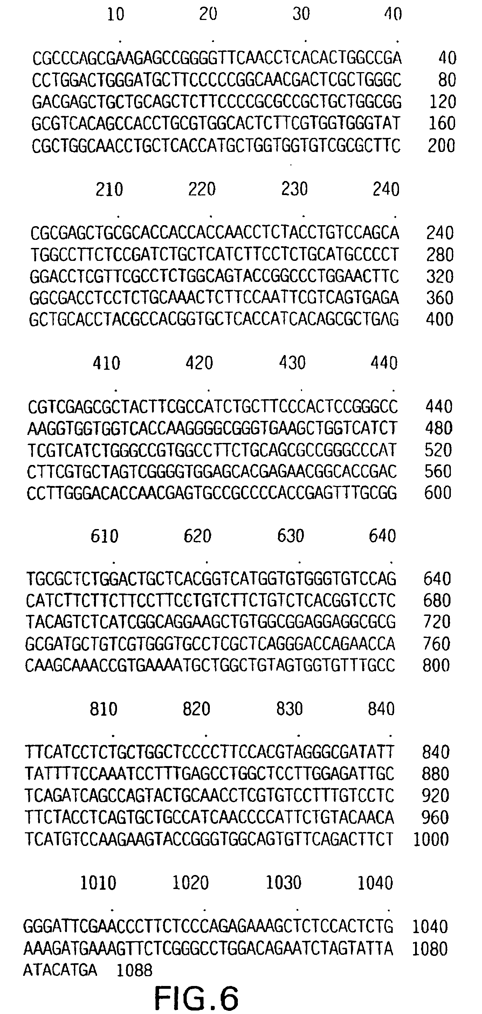

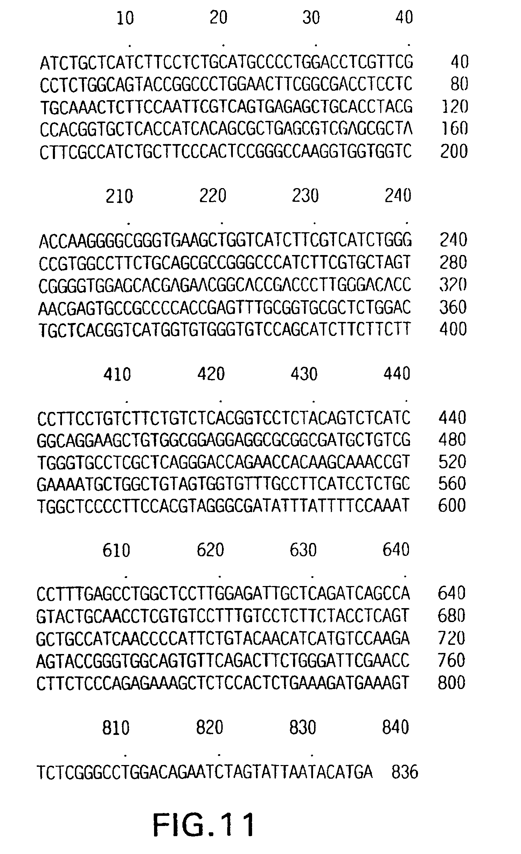

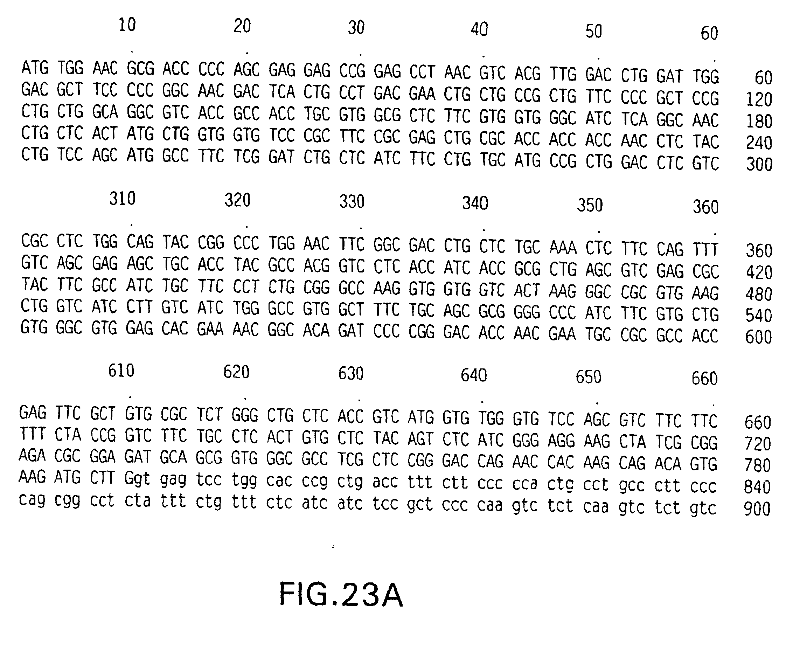

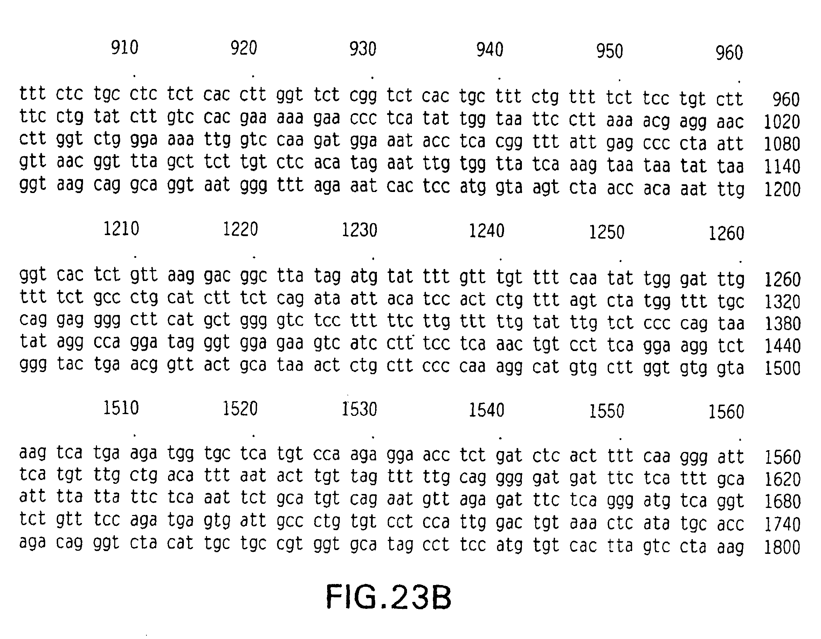

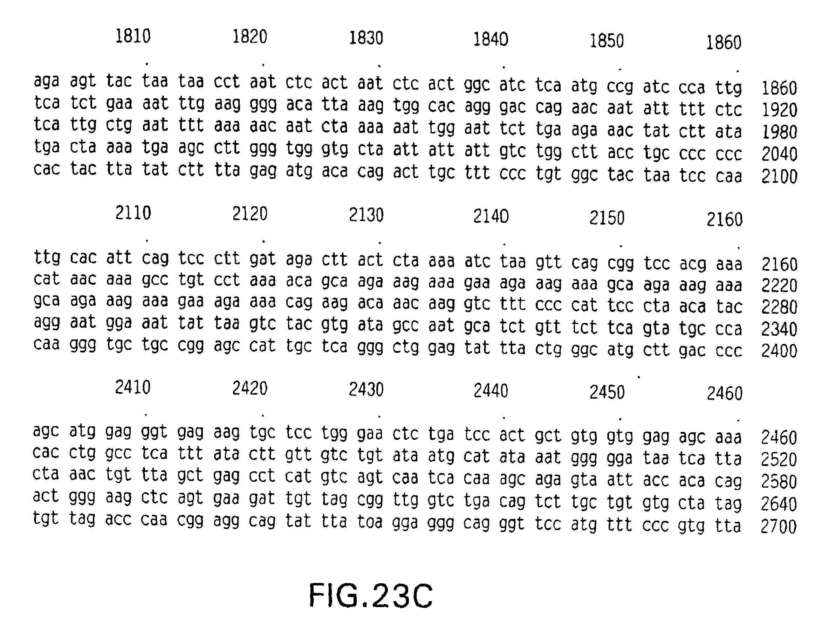

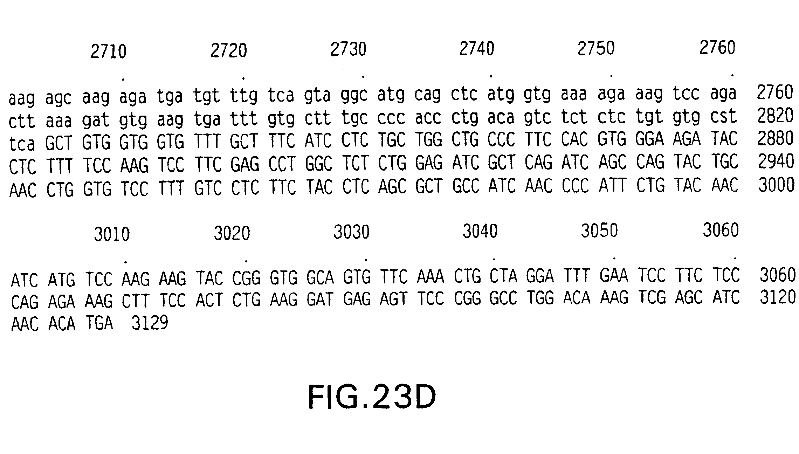

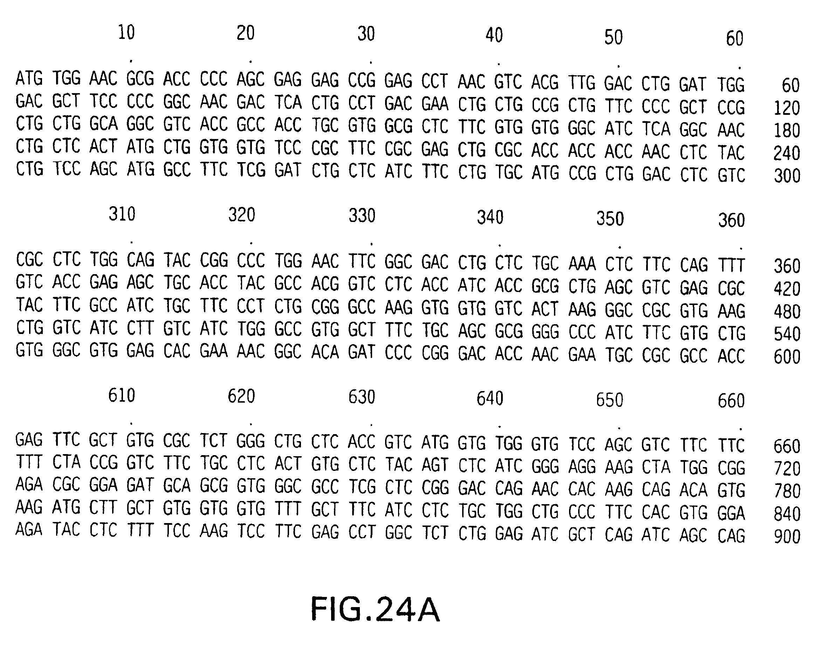

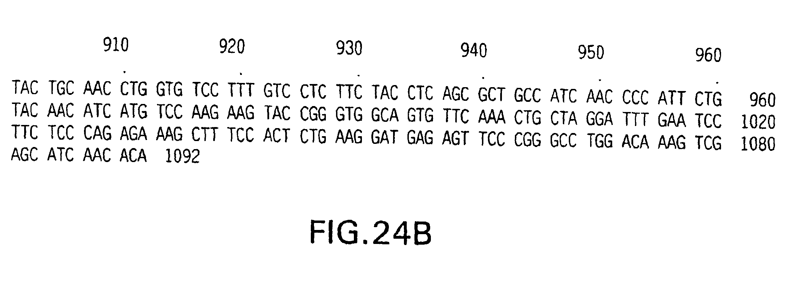

There is also provided an isolated growth hormone receptor nucleic acid which encodes an amino acid sequence as set forth in Figure 3, 7, 12, 22 or 25. A swine sequence is provided by Figure 1, human sequences by Figures 6 and 11 and rat sequences by Figures 23 and 24.

Growth hormone secretagogue receptors are proteins containing various functional domains, including one or more domains which anchor the receptor in the cell membrane, and at least one ligand binding domain. As with many receptor proteins, it is possible to modify many of the amino acids, particularly those which are not found in the ligand binding domain, and still retain at least a percentage of the biological activity of the original receptor. In accordance with this invention, it has been shown that the N-tenninal portions of the GHSR are not essential for its activation by the Growth Hormone Secretagogues (GHSs).

A further aspect of this invention are nucleic acids which encode a growth hormone secretagogue receptor from swine, human or rat. These nucleic acids may be free from associated nucleic acids, or they may be isolated or purified. For most cloning purposes, cDNA is a preferred nucleic acid, but this invention specifically includes other forms of DNA as well as RNAs which encode a GHSR or a functional equivalent.

Yet another aspect of this invention relates to vectors which comprise nucleic acids encoding a GHSR. These vectors may be comprised of DNA or RNA; for most cloning purposes DNA vectors are preferred. Typical vectors include plasmids, modified viruses, bacteriophage and cosmids, yeast artificial chromosomes and other forms of episomal or integrated DNA that can encode a GHSR. It is well within the skill of the ordinary artisan to determine an appropriate vector for a particular gene transfer or other use.

A further aspect of this invention are host cells which are transformed with a gene which encodes a growth hormone secretagogue receptor. The host cell may or may not naturally express a GHSR on the cell membrane. Preferably, once transformed, the host cells are able to express the growth hormone secretagogue receptor on the cell membrane. Depending on the host cell, it may be desirable to adapt the DNA so that particular codons are used in order to optimize expression. Such adaptations are known in the art, and these nucleic acids are also included within the scope of this invention. Generally, mammalian cell lines, such as COS, HEK-293, CHO, HeLa, NS/0, CV-1, GC, GH3 or VERO cells are preferred host cells, but other cells and cell lines such as *Xenopus* oocytes or insect cells, may also be used.

### BRIEF DESCRIPTION OF THE FIGURES

FIGURE 1 is the DNA of Swine GHSR (type I) contained in Clone 7-3.
FIGURE 2 is the amino acid sequence of swine GHSR encoded by the DNA of Figure 1.
FIGURE 3 is the entire open reading frame of the type I clone of Figure 1.
FIGURE 4 is the DNA of Swine GHSR (type II) contained in Clone 1375.
FIGURE 5 is the amino acid sequence of swine GHSR (type II) encoded by the DNA of Figure 4.
FIGURE 6 is the DNA for human GHSR (Type I) contained in Clone 1146.
FIGURE 7 is the amino acid sequence of human GHSR (type 1) encoded by the DNA of Figure 6.
FIGURE 8 is the entire open reading frame of Type I GHSR, encoded by the DNA sequence of Figure 6.
FIGURE 9 is the DNA for human GHSR (type II) contained in Clone 1141.
FIGURE 10 is the amino acid sequence of human GHSR (Type II) encoded by Clone 1141.
FIGURE 11 is the DNA for human GHSR (Type I) contained in Clone 1143.
FIGURE 12 is the amino acid sequence of human GHSR (Type 1) encoded by Clone 1143.
FIGURE 13 compares the ORF of swine Type I (lacking the MET initiator of the full length GHSR and lacking 12 additional amino acids) to the homologous domain of swine Type II receptors.
FIGURE 14 compares the homologous domain of human Type I and Type II receptors (the amino terminal sequence lacks the MET initiator and four additional amino acids).
FIGURE 15 compares the ORFs of swine Type I and human Type I receptors (the amino terminal sequence lacks the MET initiator and 12 additional amino acids).
FIGURE 16 compares full length swine Type II and human Type II receptors.
FIGURE 17 is a schematic diagram depicting the physical map of swine and human growth hormone secretagogue receptor cDNA clones.
FIGURE 18 is a graph demonstrating the pharmacology of the expressed swine and human growth hormone secretagogue receptors in *Xenopus* oocytes using the aequorin bioluminescence assay.
FIGURE 19 is a table demonstrating the pharmacology of the expressed swine and human growth hormone secretagogue receptors in *Xenopus* oocytes using the aequorin bioluminescence assay and various secretagogues.
FIGURE 20 is a graph representing the pharmacology of the pure expressed swine growth hormone secretagogue receptor in COS-7 cells using the ³⁵S-labeled Compound A binding assay.
FIGURE 21 is a table representing the competition analysis with the pure expressed swine growth hormone secretagogue receptor in COS-7 cells using the ³⁵S-labeled Compound A binding assay and various secretagogues and other G-protein coupled-receptors (GPC-Receptors) ligands in a competition assay.
FIGURE 22 is the amino acid sequence of the full length human GHSR (type I) encoded by clone 11304.
FIGURE 23 is the rat GHSR DNA sequence from the Met Initiation codon to the Stop codon. This sequence includes an intron.
FIGURE 24 is the open reading frame only of the rat GHSR of Figure 23.
FIGURE 25 is the deduced amino acid sequence of the ORF of Figure 24.
FIGURE 26 shows the expression of functional rat GHSR in transfected HEK-293 cells.

As used throughout the specification and claims, the following definitions shall apply:

Growth Hormone Secretagogue - any compound or agent that directly or indirectly stimulates or increases the release of growth hormone in an animal.

Ligands-- any molecule which binds to GHSR of this invention. These ligands can have either agonist, partial agonist, partial antagonist or antagonist activity.

Free from receptor-associated proteins-- the receptor protein is not in a mixture or solution with other membrane receptor proteins.

Free from associated nucleic acids- the nucleic acid is not covalently linked to DNA which it is naturally covalently linked in the organism's chromosome.

Isolated receptor--the protein is not in a mixture or solution with any other proteins.

Isolated nucleic acid-- the nucleic acid is not in a mixture or solution with any other nucleic acid.

Purified receptor-- the receptor is at least about 95% pure.

Purified nucleic acid-- the nucleic acid is at least about 95% pure.

Compound A -- (N-[1(R)-[(1,2-dihydro-1-methanesulfonylspiro[3H-indole-3,4'-piperidin]- 1'-yl)carbonyl]-2-(phenyl-methyloxy)ethyl]-2-amino-2-methyl propanamide, described in Patchett, 1995 *Proc*. *Natl. Acad. Sci*. 92:7001-7005.

Compound B -- 3-amino-3-methyl-N-(2,3,4,5-tetra-hydro-2-oxo-1-{[2'-1H-tetrazol-5-yl)(1,1'-biphenyl)-4-yl]methyl}-1H-benzazepin-3(R)yl-butanamide, described in Patchett, 1995 *Proc*. *Natl. Acad*. *Sci. 92*:7001-7005.

Compound C -- 3-amino-3-methyl-N-(2,3,4,5-tetrahydro-2-oxo-1-{[2'-1H-tetrazol-5-yl)(1,1'-biphenyl)-4-yl]methyl}-1H-benzazepin-3(S)yl-butanamide, described in U.S. Patent 5,206,235.

Standard or high stringency post hybridizational washing conditions -- 6 X SSC at 55°C

Moderate post hybridizational washing conditions --6 X SSC at 45°C

Relaxed post hybridizational washing conditions -- 6 X SSC at 30°C

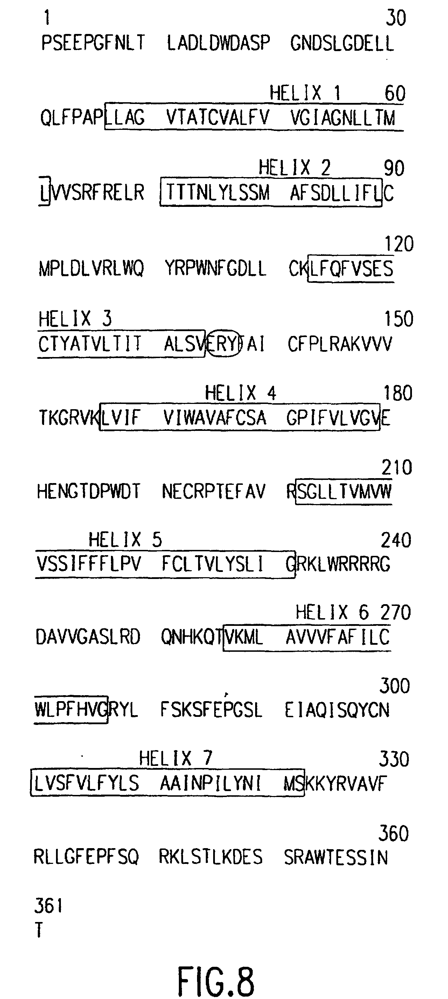

The proteins of this invention were found to have structural features which are typical of the 7-transmembrane domain (TM) containing G-protein linked receptor superfamily (GPC-R's or 7-TM receptors). Thus growth hormone secretagogue family of receptors make up new members of the GPC-R family of receptors. The intact GHSRs of this invention were found to have the general features of GPC-R's, including seven transmembrane regions, three intra- and extracellular loops, and the GPC-R protein signature sequence. The TM domains and GPC-R protein signature sequence are noted in the protein sequences of the Type I GHS receptor in Figures 3 and 8. Not all regions are required for functioning, and therefore this invention also comprises functional receptors which lack one or more non-essential domains.

The GHSRs of this invention share some sequence homology with previously cloned GPC-receptors including the rat and human neurotensin receptor (approximately 32% identity) and the rat and human TRH receptor (approximately 30% identity).

The GHSRs of this invention were isolated and characterized using expression cloning techniques in *Xenopus* oocytes. The cloning was made difficult by three factors. First, prior to this invention, there was very little information available about the biochemical characteristics and intracellular signaling/effector pathways of the proteins. Thus, cloning approaches which depended on the use of protein sequence information for the design of degenerate oligonucleotides to screen cDNA libraries or utilize PCR could not be effectively utilized. In accordance with this invention, therefore, receptor bioactivity needed to be determined.

Secondly, the growth hormone secretagogue receptor does not occur in abundance-- it is present on the cell membrane in about 10 fold less concentration than most other membrane receptors. In order to successfully clone the receptors in accordance with this invention, exhaustive precautions had be taken to ensure that the GHSR was represented in a cDNA library to be screened. This required isolation of intact, undegraded and pure poly (A)⁺ mRNA, and optimization of cDNA synthesis to maximize the production of full-length molecules. In addition, a library of larger size than normal needed to be screened (approximately 0.5 to 1 x 10⁷ clones) to increase the probability that a functional cDNA clone may be obtained.

Thirdly, no permanent cell line which expresses this receptor is known. Therefore, primary pituitary tissue had to be used as a source for mRNA or protein. This posed an additional obstacle because most primary tissues express lower amounts of a given receptor than an immortalized cell line that may be maintained in tissue culture or some tumor materials. Further, the surgical removal of a pig pituitary and extraction of biologically-active intact mRNA for the construction of a cDNA expression library is considerably more difficult than the extraction of mRNA from a tissue culture cell line. Along with the need to obtain fresh tissue continuously, there are problems associated with its intrinsic inter-animal and inter-preparation variability. The development of cell lines expressing a receptor of this invention is therefore a significant aspect of this invention.

Also disclosed is the development of an extremely sensitive, robust, reliable and high-throughput screening assay which could be used to identify portions of a cDNA library containing the receptor. This assay is described and claimed in copending patent applications Serial No. 60/008,584, filed December 13, 1995, and Attorney Docket No. 19590PV2 filed herewith.

Briefly, the ability to identify cDNAs which encode growth hormone secretagogue receptors depended upon two discoveries made in accordance with this invention: 1) that growth hormone secretagogue receptor-ligand binding events are transduced through G proteins; and 2) that a particular G protein subunit, Gₐₗₗ, must be present in the cells in order to detect receptor activity. Only when these two discoveries were made could an assay be devised to detect the presence of GHSR-encoding DNA sequences.

When the GHSR is bound by ligand (a growth hormone secretagogue), the G-proteins present in the cell activate phosphatidylinositol-specific phospholipase C (PI-PLC), an enzyme which releases intracellular signaling molecules (diacylglycerol and inositol triphosphate), which in turn start a cascade of biochemical events that promote calcium mobilization. This can be used as the basis of an assay. A detector molecule which can respond to changes in calcium concentrations, such as aequorin, a jellyfish photoprotein, is introduced into a cell along with a complex pool of up to 10,000 individual RNAs from a cDNA expression library, at least one of which may encode a GHSR. The cell is then exposed to a known growth hormone secretagogue, such as Compound A or Compound B. If one or more RNAs encodes a GHSR, then the secretagogue ligand will bind the receptor, G-protein will be activated, the calcium level will fluctuate, and the aequorin will produce measurable bioluminescence. Once a positive result is found, the procedure can be repeated with a subdivision of the RNA pool (for example, approximately 1,000, then approximately 500, then approximately 50, and then pure clones) until a single clone is identified from which RNA can be generated which encodes a GHSR.

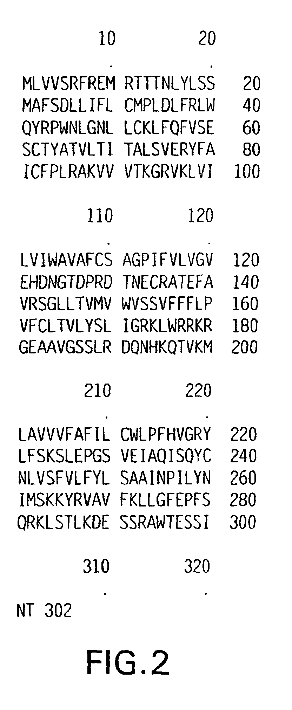

Using this general protocol in *Xenopus* oocytes with a swine cDNA expression library, Clone 7-3 was identified as containing nucleic acid encoding a swine GHSR. The insert of the cDNA clone is approximately 1.5 kb in size, and downstream from the presumed initiator methionine (MET), contains an open reading frame (ORF) encoding 302 amino acids (Mᵣ= 34,516). The DNA and deduced amino acid sequence are given in FIGURES 1 and 2. When hydropathy analysis (e.g. Kyte-Doolittle; Eisenberg, Schwartz, Komaron and Wall) is performed on the protein sequence of clone 7-3, only 6 predicted transmembrane domains are present downstream of the presumed MET initiator. Translation of the longest ORF encoded in clone 7-3 encodes a protein of 353 amino acids (Mᵣ= 39,787); however an apparent MET initiator cannot be identified for this longer reading frame (FIGURE 3). This longer reading frame is significant since 7 transmembrane segments are encoded in the 353 amino acids protein in which a MET translation initiation codon located upstream of TMl is absent. In addition, this longer protein also shares homology with known G-protein coupled receptors in its predicted TMl domain (FIGURE 3 and next sections). Thus, clone 7-3 while truncated at its amino terminus, is fully functional, demonstrating that clone 7-3 is but one embodiment of a functional equivalent of a native GHSR.

The resultant cDNA clone (or shorter portions of, for instance only 15 nucleotides long) may be used to probe libraries under hybridization conditions to find other receptors which are similar enough so that the nucleic acids can hybridize, and is particularly useful for screening libraries from other species. Using this procedure, additional human, swine, and rat GHSR cDNAs have been cloned and their nucleotide sequences determined. Further, hybridization of a cDNA to genomic DNA demonstrated that the Type I receptor (see below) is encoded by a single gene that is highly conserved. Human, monkey, rat, mouse, dog, cow, chicken and invertebrate DNA all yielded a single hybridizing species at high stringency post-hybridization conditions. Therefore, this invention is not limited to any particular species.

A swine pituitary library, a human pituitary library, and a rat pituitary library were hybridized with a radiolabeled cDNA derived from the open reading frame of the swine GHSR clone 7-3. 21 positive human GHSR cDNA clones were isolated and five swine library pools yielded a strong hybridization signal and contained clones with inserts larger than clone 7-3, as judged by their insert size on Southern blots. A single rat cDNA clone was also isolated.

Nucleotide sequence analysis revealed two types of cDNAs for both the human and swine GHSR cDNAs. The first (Type I) encodes a protein represented by clone 7-3, encoding seven transmembrane domains. The full length open reading frame appears to extend 13 amino acids beyond the largest predicted open reading frame of clone 7-3 (353 amino acids). The second (type II) diverges in its nucleotide sequence from the type I cDNA at its 3'-end, just after the predicted second amino acid of the sixth transmembrane domain (TM-6).

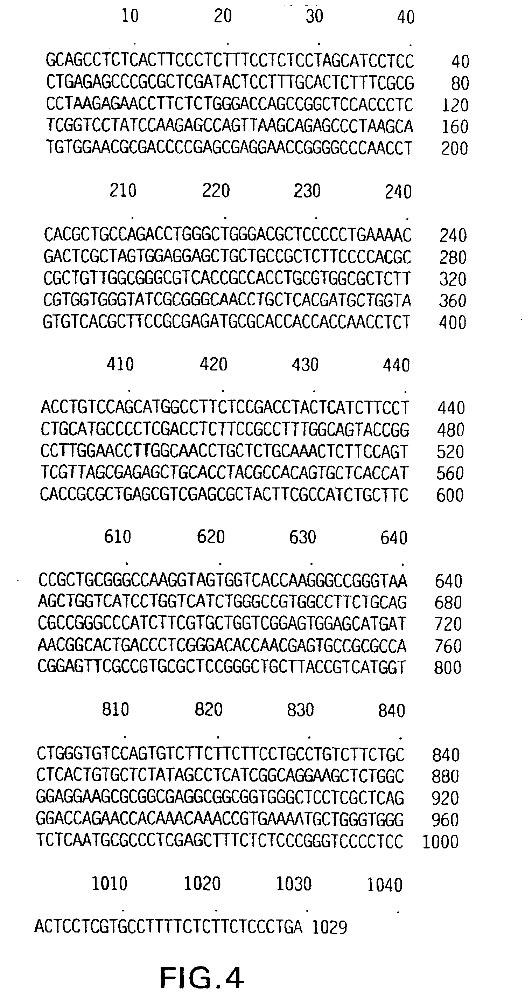

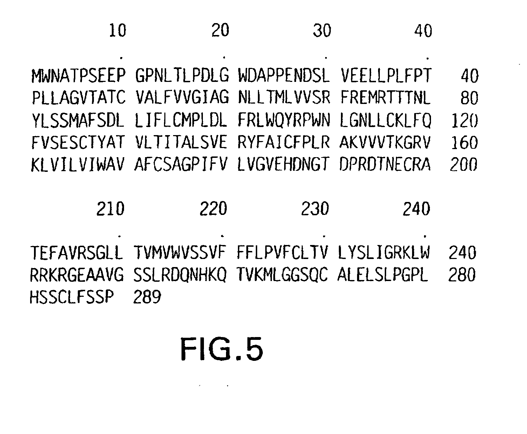

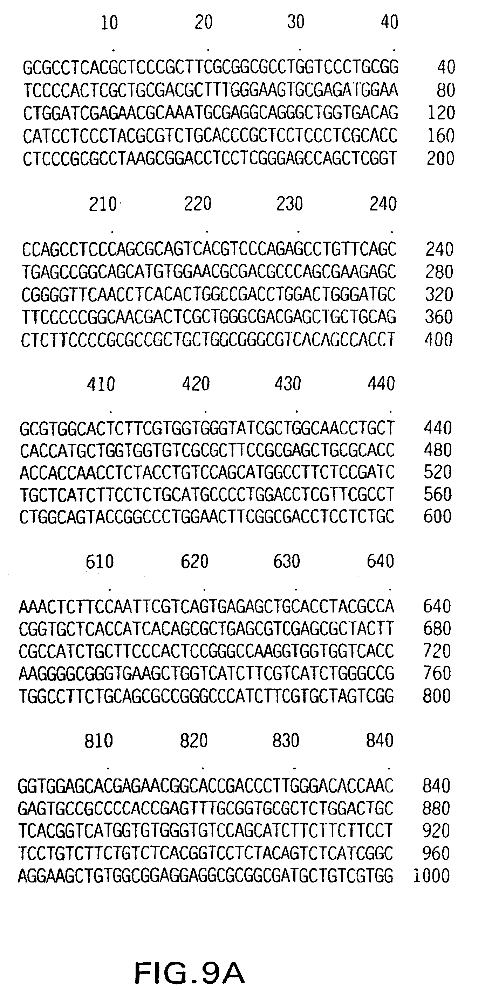

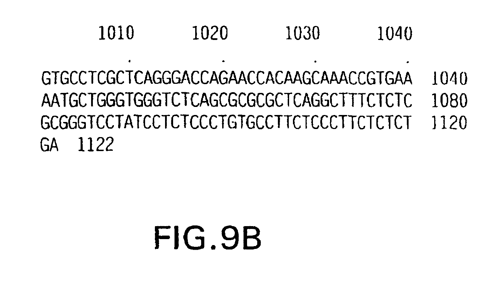

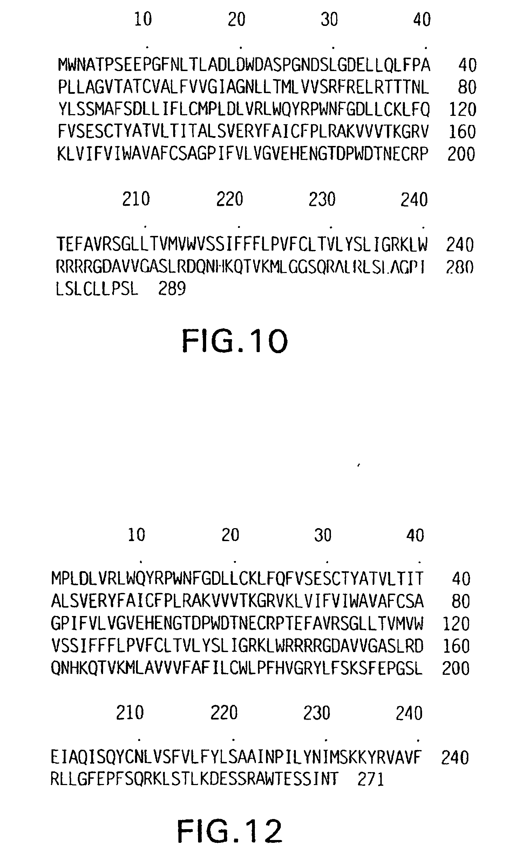

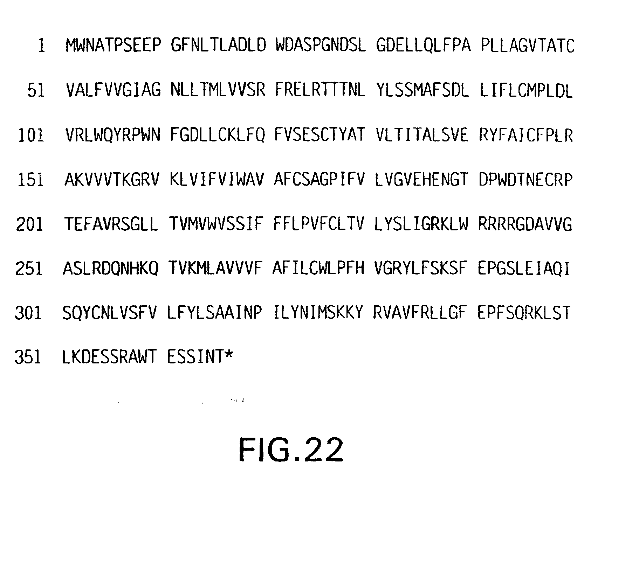

In the type II cDNAs, TM-6 is truncated and fused to a short contiguous reading frame of only 24 amino acids, followed by a translation stop codon. Swine clone 1375 is an example of a Type II cDNA (FIGURES 4 and 5). These 24 amino acids beyond TM-6 are highly conserved when compared between human and swine cDNAs. The DNA and amino acid sequences of the human GHSR Type I and II are given in FIGURES 6-12. A full length cDNA encoding the human Type I receptor, that is, a molecule encoding 7-TM domains with an initiator MET in a favorable context preceded by an inframe termination codon is isolated, and termed clone 11304. The predicted ORF of clone 11304 for the full length Type I GHSR measures 366 amino acids (Mᵣ= 41,198; FIGURE 22). The full length human Type II cDNA encodes a polypeptide of 289 amino acids (Mᵣ=32,156; FIGURES 9 and 10).

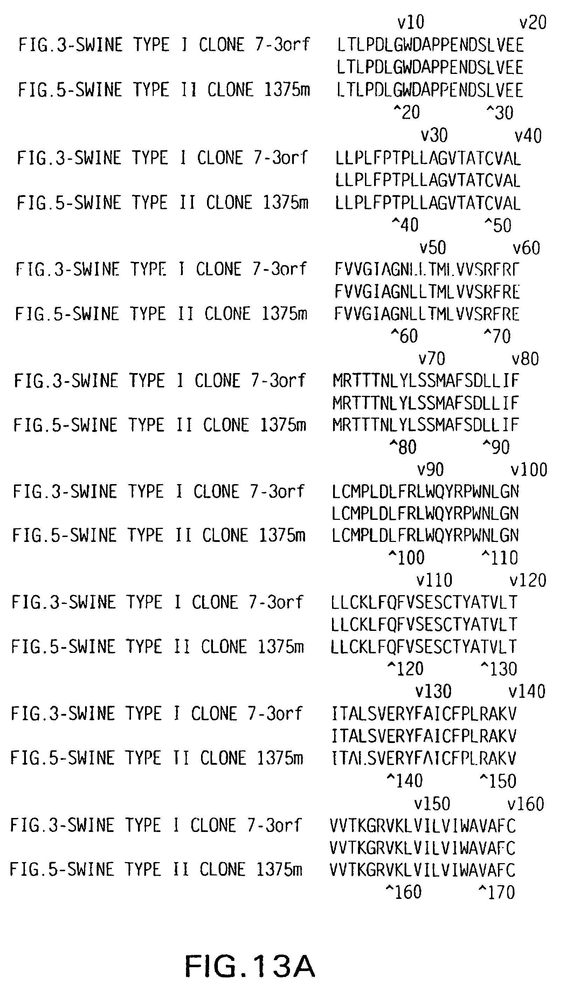

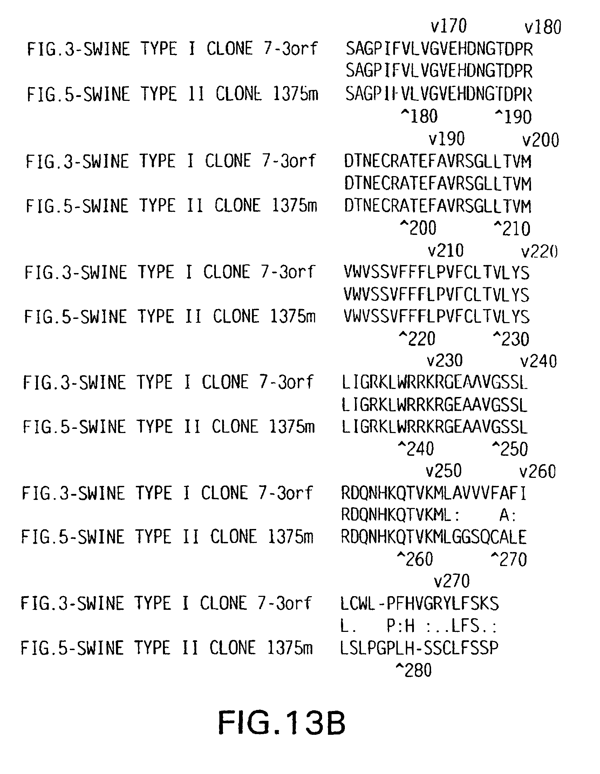

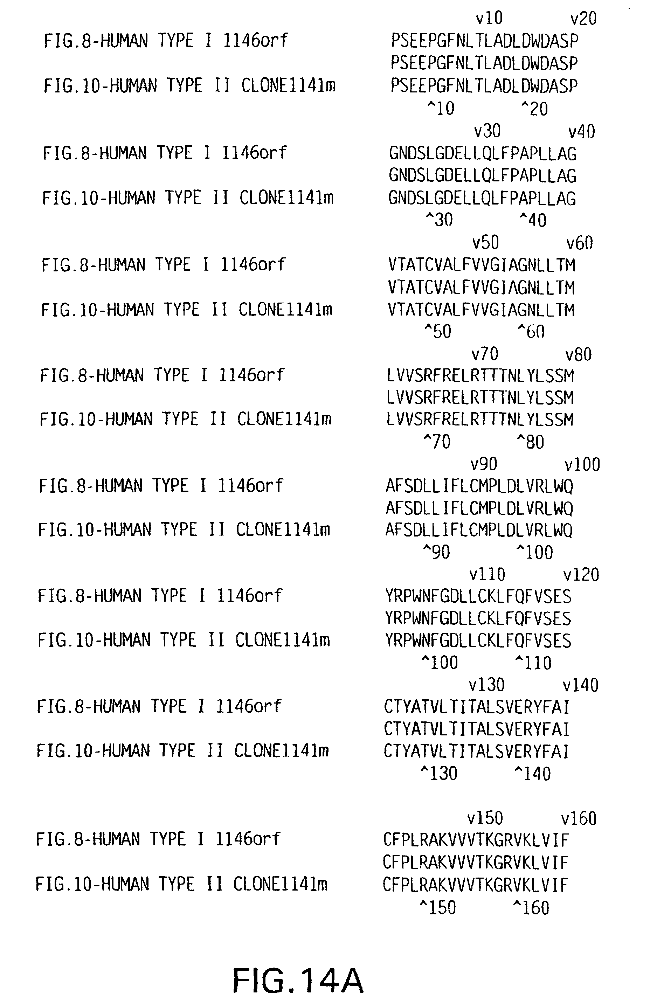

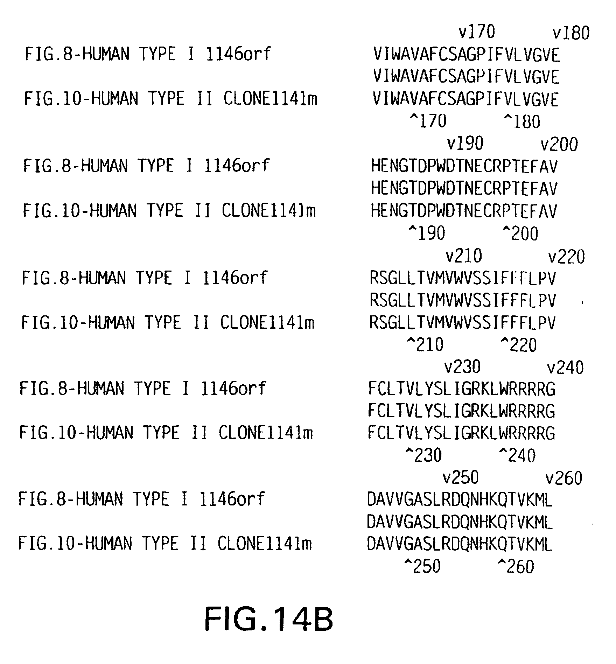

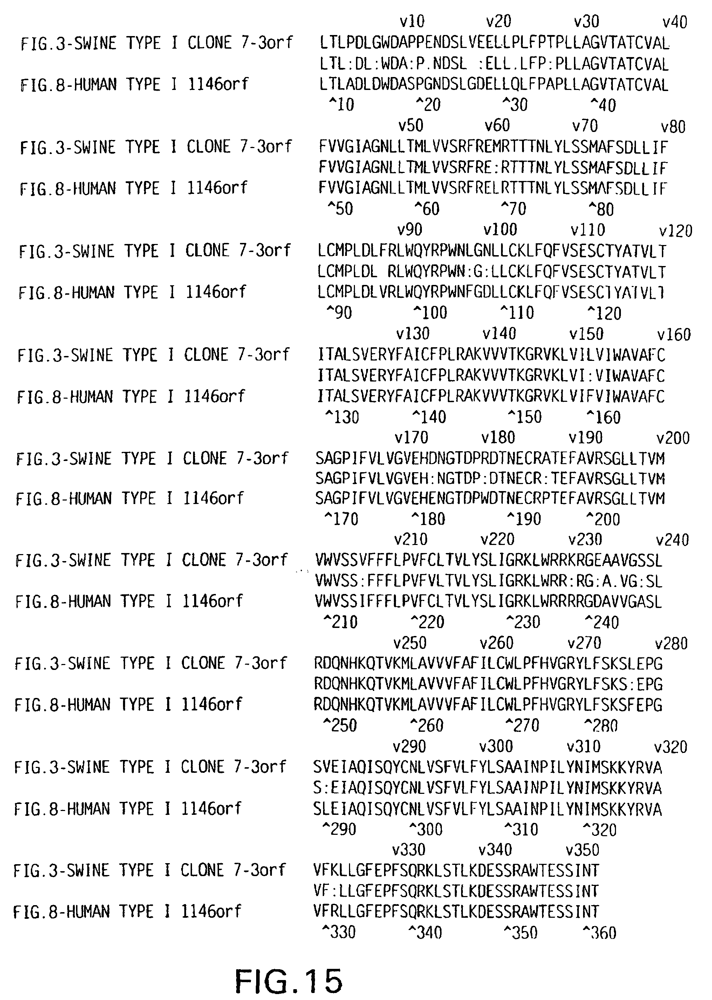

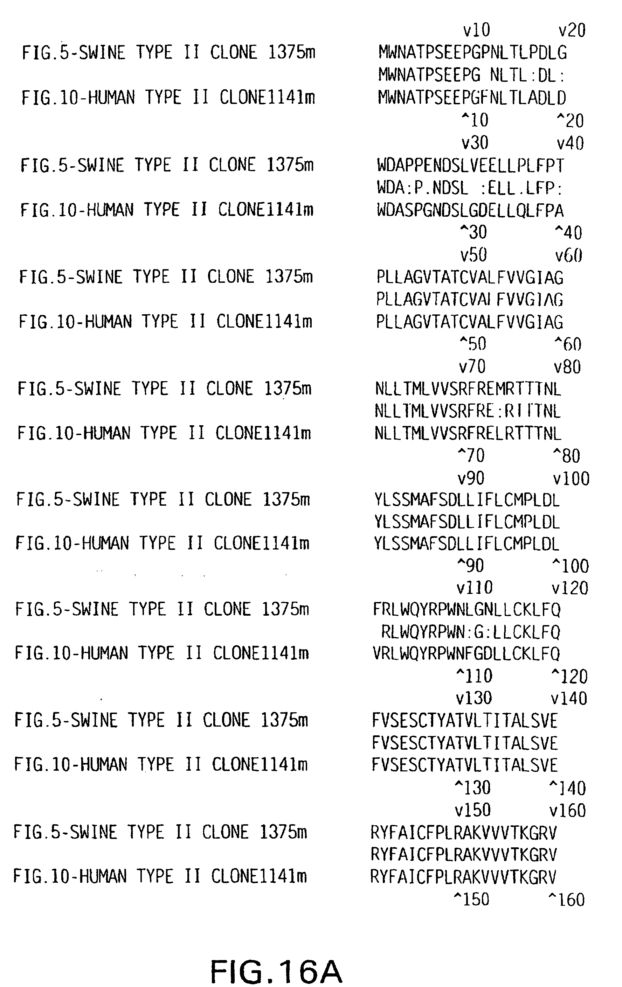

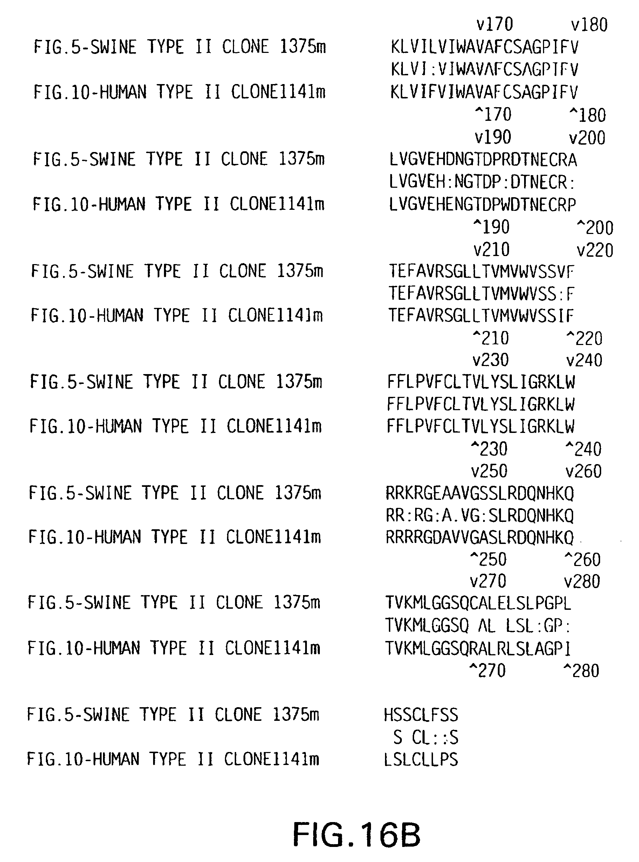

Sequence alignments performed at both the nucleic acid and protein levels show that Type I and II GHSR's are highly related to each other and across species (FIGURES 13-16). The human and swine GHSR sequences are 93% identical and 98% similar at the amino acid level.

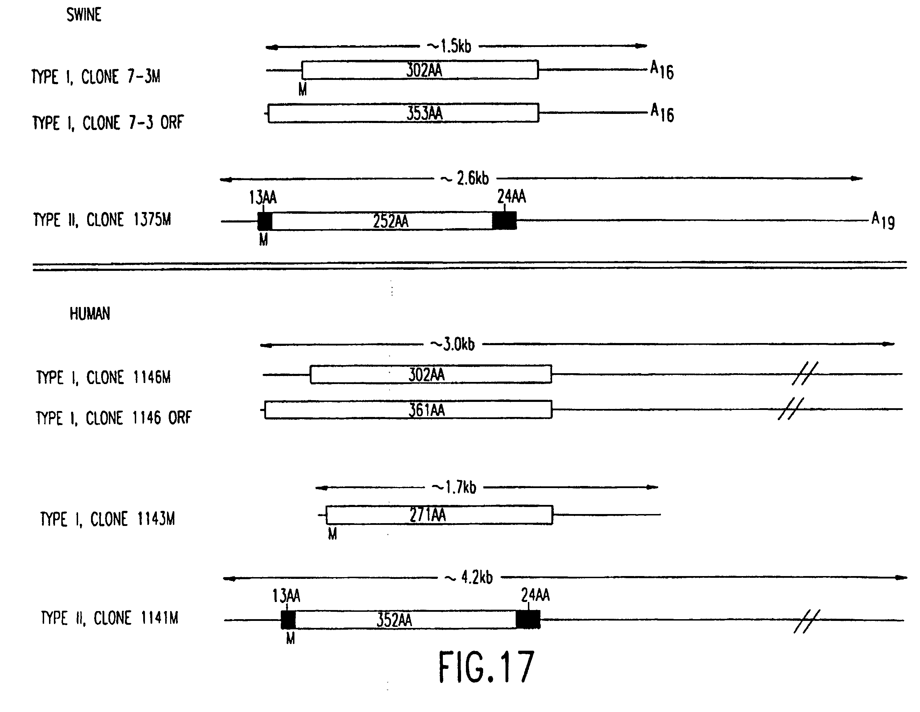

The nucleotide sequence encoding the missing amino terminal extension of swine Type I clone 7-3 is derived from the predicted full length human Type I clone and the human and swine Type II cDNAs. The reading frame of the full length clones extended 13 amino acids beyond the amino terminal sequence of clone 7-3 and this sequence was conserved in 12/13 amino acid residues, when compared between human and swine. The amino terminal extension includes a translation initiator methionine in a favorable context according to Kosak's rule. with the reading frame further upstream being interrupted by a stop codon. A schematic physical map of Type I and II swine and human cDNA clones is given in FIGURE 17.

The rat clone was also further investigated. Sequence analysis revealed the presence of a non-coding intronic sequence at nt 790 corresponding to a splice-donor site (see FIGURES 23, 24, and 25). The G/GT splice-donor site occurs two amino acids after the completion of the predicted transmembrane domain 5 (leucine 263). thus dividing the rGHSR into an amino-terminal segment (containing the extracellular domain, TM-1 through TM-5, and the first two intra- and extracellular loops) and a carboxy-terminal segment (containing TM-6, TM-7, the third intra- and extra- cellular loops, and the intra- cellular domain). The point of insertion and flanking DNA sequence are highly conserved, and also present in both human and swine Type I and II cDNAs.

Comparison of the complete open reading frame encoding the rat GHSR protein to human and swine homologs reveals a high degree of sequence identity (rat vs. human, 95.1%; rat vs. swine 93.4%.

The human GHSR can be assigned by fluorescent *in situ* hybridization analysis [FISH; as described in *Cytogenet*, *Cell Genet* 69: 196 (1995)] to the cytogenetic band 3Q26.2. The mouse gene is located on 3A3.

Human and swine Type I cRNAs expressed in oocytes were functional and responded to Compound A concentrations ranging from 1 mM to as low as 0.1 nM in the aequorin bioluminescence assay. Human or swine Type II-derived cRNAs that are truncated in TM-6 failed to give a response when injected into oocytes and these represent a receptor subtype which may bind the GHS, but cannot effectively activate the intracellular signal transduction pathway. In addition the type II receptor may interact with other proteins and thus reconstitute a functional GHSR. Proteins such as these which may have ligand-binding activity, but are not active in signal transduction are particularly useful for ligand-binding assays. In these cases, one may also over-express a mutant protein on the cell membrane and test the binding abilities of putative labeled ligands. By using a non-signaling mutant which is constitutively in a high affinity state, binding can be measured, but no adverse metabolic consequences would result. Thus non-signaling mutants are an important aspect of this invention.

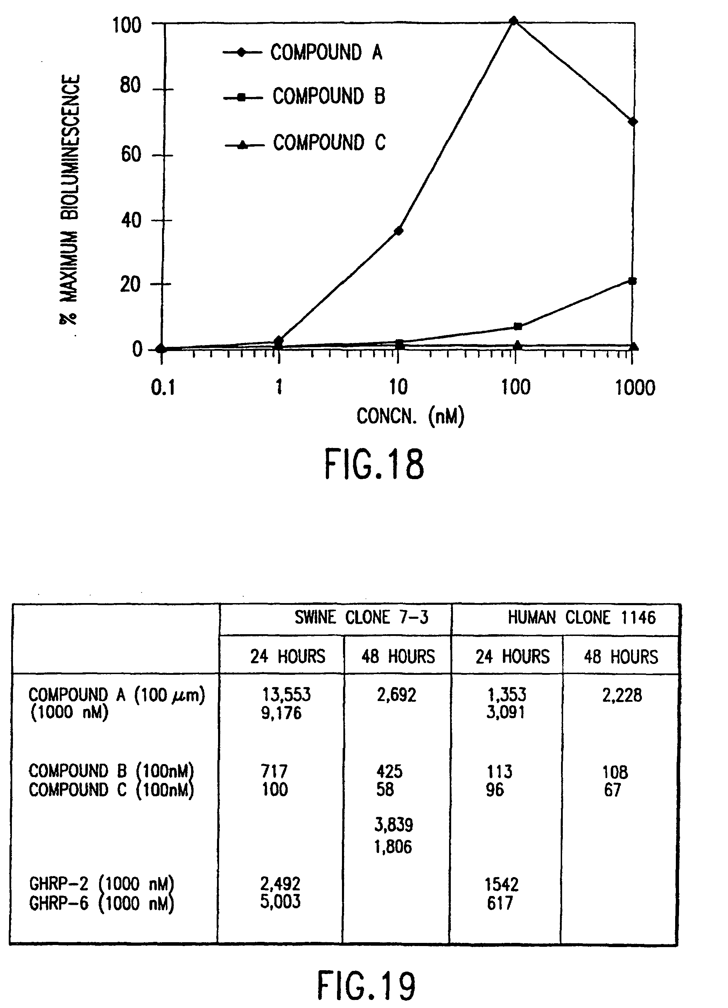

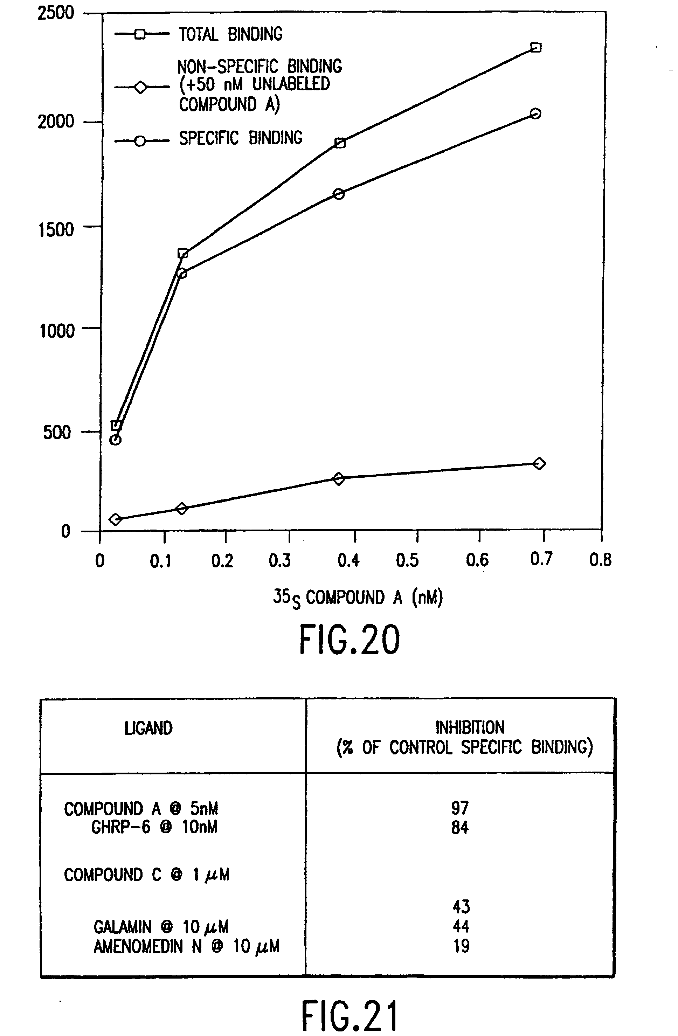

The pharmacological characterization of human, Type I swine, Type I and rat receptors in the aequorin bioluminescence assay in oocytes is summarized in FIGURES 18, 19, and 26. Peptidyl and non-peptidyl bioactive GHS's were active in a similar rank order of potency as observed for the native pituitary receptor. Independent confirmatory evidence that the Type I GHSR (shown for swine clone 7-3) encodes a fully-functional GHSR is given by the finding that when clone 7-3 is expressed transiently in mammalian COS-7 cells, high affinity (K_{D} ∼ 0.2 nM), saturable (Bₘₐₓ∼80 fmol/mg protein) and specific binding (> 90 % displaced by 50 nM unlabeled Compound A) is observed for ³⁵S-Compound A (FIGURES 20 and 21).

The GHSR receptors of this invention may be identified by hybridization of a GHSR cDNA to genomic DNA, under relaxed or moderate post hybridizational washing conditions. This analysis yields a discreet number of hybridizing bands. A suitable human genomic library which can be used in this procedure is PAC (as described in *Nature Genetics* 6:84 (1994)) and a suitable mouse genomic library is BAC (as described in *Proc Natl Acad Sci USA* 89: 8794 (1992).

Due to the high degree of homology to GHSRs, the GHSRs of this invention are believed to function similarly to GHSRs and have similar biological activity. They are useful in understanding the biological and physiological pathways involved in an organisms growth. They may be also used to scan for growth hormone secretagogue agonists and antagonists; as in particular to test the specificity of identified ligands.

Heterotrimeric G proteins, consisting of a, b and g subunits, serve to relay information from cell surface receptors to intracellular effectors, such as phospholipase C and adenylate cyclase. The G-protein alpha subunit is an essential component of the intracellular signal transduction pathway activated by receptor-ligand interaction. In the process of ligand-induced GPCR activation, the Ga subunit of a trimeric Gabg exchanges its bound GDP for GTP and dissociate from the bg heterodimer. The dissociated subunit serves as the active signal transducer, often in concert with the bg complex, thus starting the activation of the intracellular signal transduction pathway. By definition, cell surface receptors which couple intracellularly through G protein interactions are termed GPC-R's. This interaction has mainly been characterized with respect to the type of G-alpha (Gₐ) subunit which is primarily involved in the signal transduction process. Gₐ subunits are classified into sub-families based on sequence identity and the main type of effectors to which they are coupled have been characterized: Gₛ, activate adenylate cyclase; G_{i/o/t} , inhibit adenylate cyclase: Gq/ll, activate PI-PLC; and G_{12/13}, effector unknown.

Expression of several receptors in heterologous cells has been shown to be increased by the co-expression of certain Gₐ subunits. This observation formed the basis for the rationale to the use of Gₐ subunits of several sub-families in conjunction with a source of GHSR (swine poly|A⁺| mRNA) to test if a GHS-induced functional response could be measured in the *Xenopus* oocyte system. GHS-induced responses were detected and were found to be strictly dependent on Gₐₗₗ co-expression in this system, an unprecedented finding outlining the specificity of the interaction. Thus another aspect of this invention is a method of detecting a GHS response comprising co-expressing a Gₐₗₗ protein subunit in a cell also expressing a GHSR, exposing the cell to a GHS, and detecting the response.

Ligands detected using assays described herein may be used in the treatment of conditions which occur when there is a shortage of growth hormone, such as observed in growth hormone deficient children, elderly patients with musculoskeletal impairment and recovering from hip fracture, and osteoporosis.

The GHSR and fragments are immunogenic. Thus, another aspect of this invention is antibodies and antibody fragments which can bind to GHSR or a GHSR fragment. These antibodies may be monoclonal antibodies and produced using either hybridoma technology or recombinant methods. They may be used as part of assay systems or to deduce the function of a GHSR present on a cell membrane.

Also disclosed are antisense oligonucleotides nucleotides which can bind to GHSR nucleotides and modulate receptor function or expression.

Further disclosed is a method of increasing the amount of GHSRs on a cell membrane comprising, introducing into the cell a nucleic acid encoding a GHSR, and allowing expression of the GHSR.

A GHS receptor, preferably imobilized on a solid support, may be used diagnostically for the determination of the concentration of growth hormone secretagogues, or metabolites thereof, in physiological fluids, e.g., body fluids, including serum, and tissue extracts, as for example in patients who are undergoing therapy with a growth hormone secretagogue.

The administration of a GHS receptor to a patient may also be employed for purposes of: amplifying the net effect of a growth hormone secretagogue by providing increased downstream signal following administration of the growth hormone secretagogue thereby diminishing the required dosage of growth hormone secretagogue; or diminishing the effect of an overdosage of a growth hormone secretagogue during therapy.

The following Examples are presented to better illustrate the invention.

### EXAMPLE 1

### Oocyte Preparation and Selection

*Xenopus laevis* oocytes were isolated and injected using standard methods previously described by Arena, *et al*., 1991, *Mol*. *Pharmacol*. 40, 368-374, which is hereby incorporated by reference. Adult female *Xenopus laevis* frogs (purchased from Xenopus One, Ann Arbor, Ml) were anesthetized with 0.17% tricaine methanesulfonate and the ovaries were surgically removed and placed in a 60 mm culture dish (Falcon) containing OR-2 medium without calcium (82.5 mM NaCl, 2 mM KCl, 2.5 mM sodium pyruvate, 1 mM MgCl₂, 100 m/ml penicillin, 1 mg/ml streptomycin, 5 mM HEPES, pH=7.5; ND-96 from Specialty Media, NJ). Ovarian lobes were broken open, rinsed several times, and oocytes were released from their sacs by collagenase A digestion (Boehringer-Mannheim; 0.2% for 2-3 hours at 18°C) in calcium-free OR-2. When approximately 50% of the follicular layers were removed, Stage V and VI oocytes were selected and placed in ND-86 with calcium (86 mM NaCl, 2 mM KCl, 1 mM MgCl₂, 1.8 mM CaCl₂, 2.5 mM sodium pyruvate, 0.5 mM theopylline, 0.1 mM gentamycin, 5 mM HEPES (pH=7.5]). For each round of injection, typically 3-5 frogs were pre-tested for their ability to express a control G-protein linked receptor (human gonadotropin-releasing hormone receptor) and show a robust phospholipase C intracellular signaling pathway (incubation with 1% chicken serum which promotes calcium mobilization by activation of phospholipase C). Based on these results, 1-2 frogs were chosen for library pool injection (50 nl of cRNA at a concentration of 25 ng (complex pools) to 0.5 ng (pure clone) per oocyte usually 24 to 48 hours following oocyte isolation.

### EXAMPLE 2

### mRNA Isolation

Total RNA from swine (50-80 kg, Yorkshire strain) pituitaries (snap-frozen in liquid nitrogen within 1-2 minutes of animal sacrifice) was prepared by a modified phenol:guanidinium thiocyanate procedure (Chomczynski, *et al*., 1987 *Anal. Biochem*. 162:156-159, using the TRI-Reagent LS as per the manufacturer's instructions (Molecular Research Center, Cincinnati, OH). Typically, 5 mg of total RNA was obtained from 3.5 g wet weight of pituitary tissue. Poly (A)⁺ RNA was isolated from total RNA by column chromatography (two passes) on oligo (dT) cellulose (Pharmacia, Piscataway, NJ). The yield of poly (A)⁺ mRNA from total RNA was usually 0.5%. RNA from other tissues was isolated similarly.

### EXAMPLE 3

### cDNA Library Construction

First-strand cDNA was synthesized from poly (A) ⁺ mRNA using M-MLV RNAse (-) reverse transcriptase (Superscript, GIBCO-BRL, Gaithersberg, MD) as per the manufacturer's instructions with an oligo (dT)/Not I primer-adapter. Following second-strand cDNA synthesis, double-stranded cDNA was subjected to the following steps: 1) ligation to EcoR I adapters, 2) Not digestion, and 3) enrichment for large cDNAs and removal of excess adapters by gel filtration chromatography on a Sephacryl S-500 column (Pharmacia). Fractions corresponding to high molecular weight cDNA were ligated to EcoR I/Not I digested pSV-7, a eucaryotic expression vector capable of expressing cloned cDNA in mammalian cells by transfection (driven by SV-40 promoter) and in oocytes using *in vitro* transcripts (initiated from the T7 RNA polymerase promoter). pSV-7 was constructed by replacing the multiple cloning site in pSG-5 (Stratagene, La Jolla, CA; Green, S. *et al*., 1988 *Nucleic Acids Res*. 16:369), with an expanded multiple cloning site. Ligated vector:cDNA was transformed into *E*.*coli* strain DH10B (GIBCO-BRL) by electroporation with a transformation efficiency of 1 x 10⁶ pfu/10 ng double-stranded cDNA. The library contained approximately 3 x 10⁶ independent clones with greater than 95% having inserts with an average size approximating 1.65 kb (range 0.8-2.8 kb). Unamplified library stocks were frozen in glycerol at -70°C until needed. Aliquots of the library were amplified once prior to screening by a modification of a solid-state method (Kriegler, M. in *Gene Transfer and Expression: A Laboratory Manual* Stockton Press, NY 1990). Library stocks were titered on LB plates and then the equivalent of 500-1000 colonies was added to 13 ml of 2 x YT media containing 0.3% agarose and 100 mg/ml carbenicillin in a 14 ml roundbottom polypropylene tube (Falcon). The bacterial suspension was chilled in a wet ice bath for 1 hour to solidify the suspension, and then grown upright at 37°C for 24 hrs. The resultant bacterial colonies were harvested by centrifugation at 2000 x g at RT for 10 min, resuspended in 3 ml 2X YT/ carbenicillin. Aliquots were taken for frozen stocks (5%) and plasmid DNA preparation.

### EXAMPLE 4

### Plasmid DNA Preparation and cRNA Transcription

Plasmid DNA was purified from pellets of solid-state grown bacteria (1000 pools of 500 independent clones each) using the Wizard Miniprep kit according to the manufacturer's instructions (Promega Biotech, Madison, WI). The yield of plasmid DNA from a 14 ml solid-state amplification was 5-10 mg. In preparation for cRNA synthesis. 4 mg of DNA was digested with Not I, and the subsequent linearized DNA was made protein and RNase-free by proteinase K treatment (10 mg for 1 hour at 37°C), followed by two phenol, two chloroform/isoamyl alcohol extractions, and two ethanol precipitations. The DNA was resuspended in approximately 15 ml of RNase-free water and stored at -70°C until needed. cRNA was synthesized using a kit from Promega Biotech with modifications. Each 50 ml reaction contained: 5 ml of linearized plasmid (approximately 1 mg), 40 mM Tris-HCl (pH=7.5), 6 mM MgCl₂, 2 mM spermidine, 10 mM NaCl, 10 mM DTT. 0.05 mg/ml bovine serum albumin, 2 units/ml RNasin, 800 mM each of ATP, CTP and UTP, 200 mM GTP, 800 mM m7G(5')ppp(5')G, 80 units of T7 RNA polymerase, and approximately 20,000 cpm of ³²P-CTP as a trace for quantitation of synthesized RNA by TCA precipitation. The reaction was incubated for 3 hrs. at 30°C; 20 units of RNase-free DNase was added, and the incubation was allowed to proceed for an additional 15 min. at 37°C. cRNA was purified by two phenol, chloroform/isoamyl alcohol extractions, two ethanol precipitations, and resuspended at a concentration of 500 ng/ml in RNase-free water immediately before use.

### EXAMPLE 5

### Aequorin Bioluminescence Assay (ABA) and Clone Identification

The ABA requires injection of library pool cRNA (25 ng/egg for pool sizes of 500 to 10,000) with aequorin cRNA (2 ng/egg) supplemented with the G-protein alpha subunit Gₐₗₗ (2 ng/egg). To facilitate stabilization of synthetic transcripts from aequorin and Gₐₗₗ plasmids, the expression vector pCDNA-3 was modified (termed pcDNA-3v2) by insertion (in the Apa I restriction enzyme site of the polylinker) of a cassette to append a poly (A) tract on all cRNA's which initiate from the T7 RNA polymerase promoter. This cassette includes (5' to 3'): a Bgl II site, pA (20) and a Sfi I site which can be used for plasmid linearization. Polymerase chain reaction (PCR) was utilized to generate a DNA fragment corresponding to the open reading frame (ORF) of the aequorin cDNA with an optimized Kosak translational initiation sequence (Inouye, S. *et*. *al*., 1985, *Proc*. *Natl*. *Acad*. *Sci. USA* R2:3154-315R). This DNA was ligated into pCDNA-3v2 linearized with EcoR I and Kpn I in the EcoR I/Kpn I site of pCDNA-3v2. G_{al l} cDNA was excised as a Cla I/Not fragment from the pCMV-5 vector (Woon, C. *et*. *al*., 1989 *J. Biol*. *Chem*. 264: 5687-93), made blunt with Klenow DNA polymerase and inserted into the EcoR V site of pcDNA-3v2. cRNA was injected into oocytes using the motorized "Nanoject" injector (Drummond Sci. Co., Broomall, PA.) in a volume of 50 nl. Injection needles were pulled in a single step using a Flaming/Brown micropipette puller, Model P-87 (Sutter Instrument Co) and the tips were broken using 53X magnification such that an acute angle was generated with the outside diameter of the needle being <3 mm. Following injection, oocytes were incubated in ND-96 medium, with gentle orbital shaking at 18°C in the dark. Oocytes were incubated for 24 to 48 hours (depending on the experiment and the time required for expression of the heterologous RNA) before "charging'' the expressed aequorin with the essential chromophore coelenterazine. Oocytes were "charged" with coelenterazine by transferring them into 35 mm dishes containing 3 ml charging medium and incubating for 2-3 hours with gentle orbital shaking in the dark at 18°C. The charging medium contained 10 mM coelenterazine (Molecular Probes, Inc.. Eugene, OR.) and 30 mM reduced glutathione in OR-2 media (no calcium). Oocytes were then returned to ND-86 medium with calcium medium described above and incubation continued in the dark with orbital shaking until biolumineseence measurements were initiated. Measurement of GHSR expression in oocytes was performed using a Berthold Luminometer LB953 (Wallac Inc.. Gaithersburg, MD) connected to a PC running the Autolumat-PC Centrol software (Wallac Inc., Gaithersburg, MD). Oocytes (singly or in pairs) were transferred to plastic tubes (75 x 12 mm, Sarstedt) containing 2.9 ml Ca⁺⁺-free OR-2 medium. Each cRNA pool was tested using a minimum of 3 tubes containing oocytes. Bioluminescence measurements were triggered by the injection of 0.1 ml of 30 mM MK-677 (1 mM final concentration) and recordings were followed for 2 min. to observe kinetic responses consistent with an IP₃-mediated response.

Pool S10-20 was prepared from the unfractionated swine pituitary cDNA library and was composed of 10 pools each of 1000 clones. S10-20 gave a positive signal on two luminometer instruments and the component pools were then individually tested for activity. From the 10 pools of 1000 clones, only pool S271 gave a positive response. This pool was made from two pools of 500 clones designated P541 and P542. Again, only one of the pools, P541, gave a positive bioluminescent signal in the presence of 1 mM Compound A. At this point, the bacterial titer was determined in the glycerol stock of P541 such that dilutions could be plated onto LB agar plates containing 100 mg/ml carbenicillin to yield approximately 50 colonies per plate. A total of 1527 colonies were picked and replicated from 34 plates. The colonies on the original plates were then washed off, plasmids isolated, cRNA synthesized and injected into oocytes. cRNA prepared from 8 of the 34 plates gave positive signals in oocytes. Two plates were selected and the individual colonies from these plates were grown up, plasmid isolated, cRNA prepared and injected into oocytes. A single clonal isolate from each plate (designated as clones 7-3 and 28-18) gave a positive bioluminescence response to 1 mM Compound A. Clone 7-3 was further characterized.

### EXAMPLE 6

### Receptor Characterization

DNA sequencing was performed on both strands using an automated Applied Biosystems instrument (ABI model 373) and manually by the dideoxy chain termination method using Sequenase II (US Biochemical, Cleveland, OH). Database searches (Genbank 88, EMBL 42, Swiss-Prot 31, PIR 40, dEST, Prosite, dbGPCR ), sequence alignments and analysis of the GHSR nucleotide and protein sequences were carried out using the GCG Sequence Analysis Software Package (Madison, Wl; pileup, peptide structure and motif programs), PASTA and BLAST search programs, and the PC/Gene software suite from Intelligenetics (San Francisco, CA; protein analysis programs). Northern blot analysis was conducted using total (20 mg/lane) or poly (A)+ mRNA (5-10 mg/lane) prepared as described above. RNA was fractionated on a 1 % agarose gel containing 2.2 M formaldehyde and blotted to a nitrocellulose membrane. Southern blots were hybridized with a PCR generated probe encompassing the majority of the ORF predicted by clone 7-3 (nt 291 to 1132). The probe was radiolabeled by random-priming with [a]³²P-dCTP to a specific activity of greater than 10⁹ dpm/mg. Southern blots were pre-hybridized at 42°C for 4 hrs. in 5 X SSC, 5 x Denhardt's solution, 250 mg/ml tRNA, 1% glycine, 0.075% SDS, 50 mM NaPO₄ (pH 6) and 50% formamide. Hybridizations were carried out at 42°C for 20 hrs. in 5 X SSC, 1 X Denhardt's solution, 0.1% SDS, 50 mM NaPO₄, and 50% formamide. RNA blots were washed in 2 x SSC, 0.2% SDS at 42°C and at -70°C. RNA size markers were 28S and 18S rRNA and *in vitro* transcribed RNA markers (Novagen). Nylon membranes containing EcoR I and Hind III digested genomic DNA from several species (Clontech; 10 mg/lane) were hybridized for 24 hrs. at 30°C in 6 X SSPE, 10 X Denhardt's, 1% SDS, and 50% formamide. Genomic blots were washed twice with room temperature 6 X SSPE, twice with 55°C 6 X SSPE, and twice with 55°C 4 X SSPE. Additional swine GHSR clones from the swine cDNA library (described above) were identified by hybridization to plasmid DNA (in pools of 500 clones each) immobilized to nylon membranes in a slot-blot apparatus (Scheicher and Schuell). Pure clonal isolates were subsequently identified by colony hybridization. Swine GHSR clones that extend further in a 5' direction were identified using 5' RACE procedures (Frohman, M. A., 1993 *Methods Enzymol*. 218:340-358, which is incorporated by reference) using swine pituitary poly (A)⁺ mRNA as template.

### EXAMPLE 7

### Human GHSR

Human pituitary homologues of the swine GHSR were obtained by screening a commercially available cDNA library constructed in the vector lambda ZAP II (Stratagene) as per the manufacturer's instructions. Approximately 1.86 x 10⁶ phages were initially plated and screened using a random-primer labeled portion of swine clone 7-3 (described above) as hybridization probe. Twenty one positive clones were plaque purified. The inserts from these clones were excised from the bacteriophage into the phagemid pBluescript II SK- by co-infection with helper phage as described by the manufacturer (Stratagene). Human clones were characterized as has been described above for the swine clone.

### EXAMPLE 8

### Assays

Mammalian cells (COS-7) were transfected with GHSR expression plasmids using Lipofectamine (GIBCO-BRL; Hawley-Nelson, P. 1993, *Focus* 15:73). Transfections were performed in 60 mm dishes on 80% confluent cells (approximately 4 x 10⁵cells) with 8 mg of Lipofectamine and 32 mg of GHSR plasmid DNA.

Binding of ³⁵S-Compound A to swine pituitary membranes and crude membranes prepared from COS-7 cells transfected with GHSR expression plasmids was conducted. Crude cell membranes from COS-7 transfectants were prepared on ice, 48 hrs. post-transfection. Each 60 mm dish was washed twice with 3 ml of PBS, once with 1 ml homogenization buffer (50 mM Tris-HCl [pH 7.4], 5 mM MgCl₂, 2.5 mM EDTA, 30 mg/ml bacitracin). 0.5 ml of homogenization buffer was added to each dish, cells were removed by scraping and then homogenized using a Polytron device (Brinkmann, Syosset, NY; 3 bursts of 10 sec. at setting 4). The homogenate was then centrifuged for 20 min. at 11,000 x g at 0°C and the resulting crude membrane pellet (chiefly containing cell membranes and nuclei) was resuspended in homogenization buffer supplemented with 0.06% BSA (0.1 ml/60 mm dish) and kept on ice. Binding reactions were performed at 20°C for 1 hr. in a total volume of 0.5 ml containing: 0.1 ml of membrane suspension, 10 ml of ³⁵S-Compound A (0.05 to 1 nM; specific activity approximately 900 Ci/mmol), 10 ml of competing drug and 380-390 ml of homogenization buffer. Bound radioligand was separated by rapid vacuum filtration (Brandel 48-well cell harvester) through GF/C filters pretreated for 1 hr. with 0.5% polyethylenimine. After application of the membrane suspension to the filter, the filters were washed 3 times with 3 ml each of ice cold 50 mM Tris-HCl [pH 7.4], 10 mM MgCl₂, 2.5 mM EDTA and 0.015% Triton X-100, and the bound radioactivity on the filers was quantitated by scintillation counting. Specific binding (> 90% of total) is defined as the difference between total binding and non-specific binding conducted in the presence of 50 nM unlabeled Compound A.

### EXAMPLE 9

### Preparation of High Specific Activity Radioligand [³⁵S]-Compound A

[³⁵S]-Compound A was prepared from an appropriate precursor, N-[1(R)-[(1,2-dihydrospiro[3H-indole-3,4'-piperidin]-1'-yl)-carbonyl]-2-(phenyl-methyloxy)ethyl]-2-amino-t-butoxycarbonyl-2-methylpropan-amide, using methane [³⁵S]sulfonyl chloride as described in Dean DC, *et al*., 1995, In: Allen J, Voges R (eds) Synthesis and Applications of Isotopically Labelled Compounds, John Wiley & Sons, New York, pp. 795-801. Purification by semi-preparative HPLC (Zorbax SB-phenyl column, 68% MeOH/water, 0.1% TFA, 5 ml/min) was followed by N-t-BOC cleavage using 15% trifluro-acetic acid in dichloromethane (25°C, 3 hr) to give [methylsulfonyl-³⁵S]Compound A in near quantitative yield. HPLC purification (Hamilton PRP-1 4.6x250 mm column, linear gradient of 50-75% methanol-water with 1 mM HCl over 30 min. 1.3 ml/min) provided the ligand in >99% radiochemical purity. The structure was established by HPLC coelution with unlabeled Compound A and by mass spectral analysis. The latter method also indicated a specific activity of ∼1000 Ci/mmol.

### EXAMPLE 10

### DNA Encoding a Rat Growth Hormone Secretagogue Receptor (GHSR) Type la

Cross-hybridization under reduced stringency was the strategy utilized to isolate the rat GHSR type Ia. Approximately 10⁶ phage plaques of a once-amplified rat pituitary cDNA library in lambda gtll (RL1051b; Clontech, Palo Alto, CA) were plated on *E. coli* strain Y 1090r-. The plaques were transferred to maximum-strength Nytran (Schleicher & Schuell, Keene, NH) denatured, neutralized and screened with a 1.6 kb EcoRI/Notl fragment containing the entire coding and untranslated regions of the swine GHSR, clone 7-3. The membranes were incubated at 30°C in prehybridization solution (50% formamide, 2 X Denhardts, 5 X SSPE, 0.1% SDS, 100 mg/ml salmon sperm DNA) for 3 hours followed by overnight incubation in hybridization solution (50% formamide, 2 X Denhardts, 5 X SSPE, 0.1 % SDS, 10% dextran sulfate, 100 mg/ml salmon sperm DNA) with 1 x 10⁶ cpm/ml of [³²P]-labeled probe. The probe was labeled with [³²P]dCTP using a random priming kit (Gibco BRL, Gaithersburg, ND). After hybridization the blots were washed two times each with 2 X SSC, 0.1% SDS (at 24°C, then 37°C, and finally 55°C). A single positive clone was isolated following three rounds of plaque purification. Phage containing the GHSR was eluted from plate plaques with 1 x lambda buffer (0.1 M NaCl, 0.01M MgSO₄•7H₂O, 35mM Tris-HCl, pH 7.5) following overnight growth of approximately 200 pfu/150mm dish. After a ten minute centrifugation at 10,000 x g to remove debris, the phage solution was treated with 1 mg/ml RNAse A and DNAse I for thirty minutes at 24°C, followed by precipitation with 20% PEG (8000)/2M NaCl for two hours on ice, and collection by centrifugation at 10,000 x g for twenty minutes. Phage DNA was isolated by incubation in 0.1% SDS, 30mM EDTA, 50 mg/ml proteinase K for one hour at 68°C, with subsequent phenol (three times) and chloroform (twice) extraction before isopropanol precipitation overnight. The GHSR DNA insert (∼6.4 kb) was sub-cloned from lambda gt11 into the plasmid vector Litmus 28 (New England Biolabs, Beverly, MA). 2 mg of phage DNA was heated to 65°C for ten minutes, then digested with 100 units BsiWI (New England Biolab, Bevely, MA) at 37°C overnight. A 6.5 kb fragment was gel purified, electroeluted and phenol/chloroform extracted prior to ligation to BsiWI-digested Litmus 28 vector.

Double-stranded DNA was sequenced on both strands on a ABI 373 automated sequencer using the ABI PRISM dye termination cycle sequencing ready reaction kit (Perkin Elmer; Foster City, CA).

Comparison of the complete ORF encoding the rat GHSR type Ia protein sequence to human and swine GHSR homologs reveals a high degree of sequence identity (rat *vs*. human, 95.1 %; rat *vs*. swine 93.4 %).

For sequence comparisons and functional expression studies, a contiguous DNA fragment encoding the complete ORF (devoid of intervening sequence) for the rat GHSR type Ia was generated. The PCR was utilized to synthesize a amino-terminal fragment from Met-1 to Val-260 with EcoRl (5') and Hpal (3') restriction sites appended, while a carboxyl-terminal fragment was generated from Lys-261 to Thr-364 with Dra I (5') and Not I (3') restriction sites appended. The ORF construct was assembled into the mammalian expression vector pSV7 via a three-way ligation with EcoRI/Not I-digested pSV7. EcoRI/Hpa I-digested NH₂-terminal fragment, and Dra I/Not I-digested C-terminal fragment.

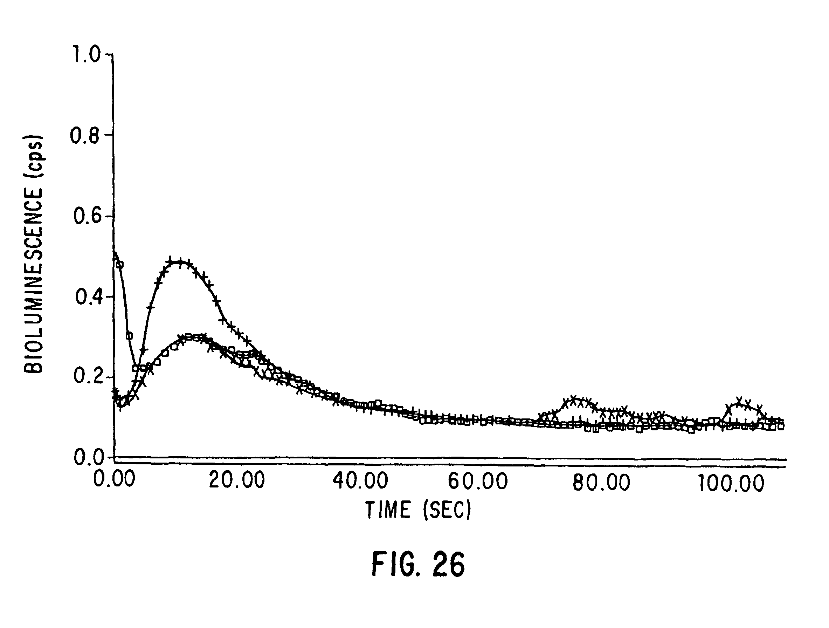

Functional activity of the ORF construct was assessed by transfecting (using lipofectamine; GIBCO/BRL) 5 mg of plasmid DNA into the aequorin expressing reporter cell line (293-AEQ17) cultured in 60 mm dishes. Following approximately 40 hours of expression the aequorin in the cells was charged for 2 hours with coelenterazine, the cells were harvested, washed and pelleted by low speed centrifugation into luminometer tubes. Functional activity was determined by measuring Compound A dependent mobilization of intracellular calcium and concomitant calcium induced aequorin bioluminescence. Shown in Fig. 26 are three replicate samples exhibiting Compound A-induced luminescent responses.

## Claims

1. An isolated growth hormone secretagogue receptor which comprises an amino acid sequence as set forth in Figure 3, 7, 12, 22 or 25.

2. A growth hormone secretagogue receptor, free from receptor-associated proteins, which comprises an amino acid sequence as set forth in Figure 3, 7, 12, 22 or 25.

3. A receptor according to claim 1 or 2 which is swine and comprises the sequence set forth in Figure 3.

4. A receptor according to claim 1 or 2 which is human and comprises the sequence set forth in Figure 7, 12 or 22.

5. A receptor according to claim 1 or 2 which is rat and comprises the sequence set forth in Figure 25.

6. An isolated growth hormone receptor nucleic acid which encodes an amino acid sequence as set forth in Figure 3, 7, 12, 22 or 25.

7. A swine nucleic acid according to claim 6 which comprises the nucleotide sequence set forth in Figure 1.

8. A human nucleic acid according to claim 6 which comprises a nucleotide sequence set forth in Figure 6 or 11.

9. A rat nucleic acid according to claim 6 which comprises a nucleotide sequence set forth in Figure 23 or 24.

10. A vector comprising a nucleic acid as defined in any one of claims 6 to 9.

11. A vector according to claim 10 which is a plasmid, modified virus, yeast artificial chromosome, bacteriophage, cosmid or transposable element.

12. A host cell comprising a vector as defined in claim 10 or 11.

## Patentansprüche

1. Isolierter Wachstumshormon-Sekretagogum-Rezeptor, welcher eine Aminosäuresequenz wie in Fig. 3, 7, 12, 22 oder 25 angegeben umfaßt.

2. Wachstumshormon-Sekretagogum-Rezeptor, frei von rezeptor-assoziierten Proteinen, welcher eine Aminosäuresequenz wie in Fig. 3, 7, 12, 22 oder 25 angegeben umfaßt.

3. Rezeptor nach Anspruch 1 oder 2, welcher ein Schweine-Rezeptor ist und die in Fig. 3 angegebene Sequenz umfaßt.

4. Rezeptor nach Anspruch 1 oder 2, welcher ein humaner Rezeptor ist und die in Fig. 7, 12 oder 22 angegebene Sequenz umfaßt.

5. Rezeptor nach Anspruch 1 oder 2, welcher ein Ratten-Rezeptor ist und die in Fig. 25 angegebene Sequenz umfaßt.

6. Isolierte Wachstumshormon-Rezeptor-Nukleinsäure, welche für eine Aminosäuresequenz wie in Fig. 3, 7, 12, 22 oder 25 angegeben kodiert.

7. Schweine-Nukleinsäure nach Anspruch 6, weiche die in Fig. 1 angegebene Nukleotidsequenz umfaßt.

8. Humane Nukleinsäure nach Anspruch 6, welche eine in Fig. 6 oder 11 angegebene Nukleotidsequenz umfaßt.

9. Ratten-Nukleinsäure nach Anspruch 6, welche eine in Fig. 23 oder 24 angegebene Nukleotidsequenz umfaßt.

10. Vektor, umfassend eine Nukleinsäure wie in irgendeinem der Ansprüche 6 bis 9 definiert.

11. Vektor nach Anspruch 10, welcher ein Plasmid, modifiziertes Virus, künstliches Hefechromosom, Bakteriophage, Cosmid oder Transposon ist.

12. Wirtszelle, umfassend einen Vektor wie in Anspruch 10 oder 11 definiert.

## Revendications

1. Récepteur sécrétagogue d'hormones de croissance isolé, qui comprend une séquence d'acides aminés telle que représentée sur les figures 3, 7, 12, 22 ou 25.

2. Récepteur sécrétagogue d'hormones de croissance dépourvu de protéines associées au récepteur, qui comprend une séquence d'acides aminés telle que représentée sur les figures 3, 7, 12, 22 ou 25.

3. Récepteur selon la revendication 1 ou 2, qui provient d'un cochon et qui comprend la séquence représentée sur la figure 3.

4. Récepteur selon la revendication 1 ou 2, qui est humain et qui comprend la séquence représentée sur les figures 7, 12 ou 22.

5. Récepteur selon la revendication 1 ou 2, qui provient d'un rat et qui comprend la séquence représentée sur la figure 25.

6. Acide nucléique isolé d'un récepteur d'hormones de croissance, qui code pour une séquence d'acides aminés telle que représentée sur les figures 3, 7, 12, 22 ou 25.

7. Acide nucléique de cochon selon la revendication 6, qui comprend la séquence de nucléotides représentée sur la figure 1.

8. Acide nucléique humain selon la revendication 6, qui comprend une séquence de nucléotides représentée sur la figure 6 ou 11.

9. Acide nucléique de rat selon la revendication 6, qui comprend une séquence de nucléotides représentée sur la figure 23 ou 24.

10. Vecteur comprenant un aide nucléique tel que défini selon l'une quelconque des revendications 6 à 9.

11. Vecteur selon la revendication 10 qui est un plasmide, un virus modifié, un chromosome artificiel de levure, un bactériophage, un cosmide ou un élément transposable.

12. Cellule hôte comprenant un vecteur tel que défini selon la revendication 10 ou 11.
